# EUROPEAN PATENT SPECIFICATION

(11) **EP 4 371 518 B1**
(45) Date of publication and mention of the grant of the patent: **02.12.2026**
(21) Application number: 22842509.6
(22) Date of filing: 15.07.2022
(51) Int. Cl.: A61B 34/00, A61B 34/30, A61B 17/00, A61B 17/29, A61B 17/32, B25J 9/10, B25J 9/16, A61B 1/00

(54) **MOVING DEVICE**
BEWEGUNGSVORRICHTUNG
DISPOSITIF DE DÉPLACEMENT

(30) Priority: 15.07.2021 KR 20210092856
(43) Date of publication of application: 22.05.2024
(60) Divisional application: 26164505.5 / 4 755 285
(73) Proprietor: Endorobotics Co., Ltd., Seoul 02841 (KR)
(72) Inventor: KIM, Kyungnam, Seoul 02858 (KR); KIM, Byung Gon, Seoul 02583 (KR)
(74) Representative: Maiwald GmbH
(86) International application number: PCT/KR2022/010364
(87) International publication number: WO 2023/287247

(56) References cited:
- JP-A- 2004 160 083
- JP-A- 2006 068 893
- KR-A- 20160 008 169
- KR-B1- 101 750 628
- KR-B1- 101 829 530
- KR-B1- 102 165 413
- US-A1- 2005 137 453
- US-A1- 2005 250 989
- US-A1- 2010 274 080

## Description

### [Technical Field]

The present invention relates to a moving device, and more specifically, to a moving device capable of moving a predetermined member using a tendon and a sheath.

### [Background Art]

US 2010/274080 A1 describes a system for performing a minimally invasive surgical procedure including a cannula, a surgical instrument for use through the cannula, and a remote positioning system to adjust the relative position between the surgical instrument and the cannula. By allowing a physician to perform the minimally invasive surgical procedure at a distance from the patient, the remote positioning system minimizes the radiation exposure of the physician while still providing accurate control over the procedure.

As one method of a control interface for remotely controlling a surgical robot or controlling an endoscope, there is a method of directly attaching an actuator to a link structure.

However, this method has a disadvantage that the actuator itself is very heavy. In order to resolve this, a cable driving method of transmitting a force generated by an actuator, which is fixed externally, through a flexible cable has been developed.

As an example of the cable driving method, technology using a tendon and a sheath has been developed. The tendon and the sheath have a thin and long shape and have been installed and used in a system in which power is transmitted through a narrow passage. In particular, the tendon is a thin, long, and flexible member. Since the tendon is prone to buckling when pressed, the tendon is configured to transmit a force by tension pulling one end portion thereof.

Conventionally, the tendon-sheath driving device for rotating a rotational link about a predetermined axis by fixing one end portion of the tendon to an outer circumferential surface of a rotational link and then pulling the other end portion using an actuator or the like in consideration of the characteristics of the tendon has been developed.

However, the conventional tendon-sheath driving device can only rotate one member about a rotational axis and cannot linearly move the one member. Therefore, there has been a demand for the development of a moving device using a tendon and a sheath, which can linearly move one member.

### [Disclosure]

### [Technical Problem]

The present invention has been devised in consideration of the above point and is directed to providing a moving device capable of moving one member relative to the other member.

The present invention is also directed to providing a moving device capable of accommodating one member in an internal space of the other member to protect the one member from the outside.

The present invention is also directed to providing a moving device capable of linearly moving one member relative to the other member.

The present invention is also directed to providing a moving device capable of rotating one member about a predetermined axis relative to the other member.

The present invention is defined by the subject-matter of claim 1.

### [Technical Solution]

According to one aspect of the present invention, there is provided a moving device including a stationary member, a first tendon of which one side is coupled to the stationary member, a first movable member that is movable with respect to the stationary member, a first sheath of which one side is coupled to the first movable member and which is formed to allow the first tendon to pass therethrough, a first holder configured to fix any one of the other side of the first tendon and the other side of the first sheath, and a first driving unit configured to move the other one of the other side of the first tendon and the other side of the first sheath, wherein the first movable member is formed to relatively move with respect to the stationary member according to relative movement of the first tendon and the first sheath.

The moving device further includes a second tendon of which one side is coupled to the first movable member, a second sheath of which one side is coupled to the stationary member, a second holder configured to fix any one of the other side of the second tendon and the other side of the second sheath, and a second driving unit configured to move the other one of the other side of the second tendon and the other side of the second sheath, wherein the first movable member is formed to relatively move with respect to the stationary member according to relative movement of the second tendon and the second sheath.

In this case, the first holder may be formed to fix the other side of the first sheath, and the first driving unit may be formed to move the other side of the first tendon.

In this case, when the first driving unit pulls the other side of the first tendon, relative movement between the stationary member and the first movable member may be caused as the one side of the first tendon is inserted into the one side of the first sheath, and the other side of the first tendon is drawn out from the other side of the first sheath.

In this case, the first holder may be formed to fix the other side of the first sheath, and the first driving unit may be formed to move the other side of the first sheath.

In another example, one side portion of the first tendon may include a first portion of which one side is coupled to the stationary member and the other side extends in a first direction and a second portion extending in a second direction from the first portion, and the first movable member may be formed to relatively move in a direction parallel to the first direction with respect to the stationary member according to the relative movement of the first tendon and the first sheath.

In this case, the one side portion of the first sheath may be disposed to be curved at a predetermined curvature so that an end portion thereof faces the direction parallel to the first direction.

In this case, the stationary member and the first movable member may each have a cylindrical shape and may be coaxially arranged, the first direction may be a circumferential direction of the stationary member, the second direction may be a direction parallel to a longitudinal direction of the stationary member, and the first movable member may be formed to relatively rotate with respect to the stationary member about the coaxial axis according to the relative movement of the first tendon and the first sheath.

In this case, the moving device may further include a second tendon of which one side is coupled to the stationary member, a second sheath of which one side is coupled to the first movable member and which is formed to allow the second tendon to pass therethrough, a second holder configured to fix any one of the other side of the second tendon and the other side of the second sheath, and a second driving unit configured to move the other one of the other side of the second tendon and the other side of the second sheath, wherein one side portion of the second tendon may include a first portion of which one side is coupled to the stationary member and the other side extends in a third direction opposite to the first direction and a second portion extending in the second direction from the first portion, and the first movable member may be formed to relatively rotate with respect to the stationary member about the coaxial axis according to relative movement between the second tendon and the second sheath.

In this case, the moving device may further include a third tendon of which one side is coupled to the first movable member, a second movable member coupled to the stationary member to relatively rotate with respect to the stationary member in a direction parallel to the first direction, a third sheath of which one side is coupled to the second movable member, a third holder configured to fix any one of the other side of the third tendon and the other side of the third sheath, and a third driving unit configured to move the other one of the other side of the third tendon and the other side of the third sheath, wherein the first movable member and the second movable member may be formed to relatively rotate with respect to the stationary member about the coaxial axis according to the relative movement between the first tendon and the first sheath, and the first movable member may be formed to relatively move in the second direction with respect to the stationary member and the second movable member according to relative movement between the third tendon and the third sheath.

In this case, one side portion of the first sheath may include a first portion formed to be curved outward from the first movable member as the first movable member moves in the second direction and a second portion coupled to the first movable member.

In this case, a stopper configured to restrict relative movement between the first movable member and the second movable member may be provided on the second movable member when the first movable member reaches a predetermined position.

In this case, the second movable member may include a body part rotatably coupled to the stationary member and an accommodating part provided at one side of the body part and accommodating the first movable member therein, and a guide hole configured to guide the first movable member to move in a front-rear direction may be formed in the accommodating part.

In the present invention, the first driving unit may include an actuator or an operating member manipulated manually.

In this case, the first driving unit may include a guide member configured to guide the other one of the other side of the first tendon and the other side of the first sheath.

In this case, the second sheath is formed to allow the second tendon to pass therethrough, wherein the first movable member is formed to relatively move with respect to the stationary member between the one side of the first tendon coupled to the stationary member and the one side of the second sheath coupled to the stationary member.

In this case, the first tendon and the second tendon may be disposed parallel to each other in a direction of relative movement between the first movable member and the stationary member.

In this case, the one side of the first tendon coupled to the stationary member may be positioned in front of the first movable member when viewed in a moving direction of the first movable member, and the one side of the second sheath coupled to the stationary member may be positioned behind the first movable member when viewed in the moving direction of the first movable member.

In this case, the stationary member may be fixedly positioned on a front end portion of an endoscope device.

In this case, a surgical member for an endoscope device may be coupled to the first movable member.

### [Advantageous Effects]

In a moving device according to one embodiment of the present invention, it is possible to cause relative movement between a stationary member and a first movable member by causing relative movement between a tendon coupled to the stationary member and a sheath which is coupled to the first movable member and through which the tendon passes.

In addition, in the moving device according to the embodiment of the present invention, since the first movable member is accommodated inside a guide hole formed in the stationary member or a second movable member and the first movable member moves relatively in an extending direction inside the guide hole, the first movable member can be protected from the outside.

In addition, since the tendon coupled to the stationary member or the second movable member can be disposed to pass through the sheath coupled to the first movable member and a driving unit can pull the tendon rearward or push the sheath forward, the moving device according to the embodiment of the present invention can move the first movable member forward relative to the stationary member or the second movable member.

In addition, since the tendon coupled to the first movable member can be disposed to pass through the sheath coupled to the stationary member or the second movable member and the driving unit can pull the tendon rearward, the moving device according to the embodiment of the present invention can move the first movable member rearward relative to the stationary member or the second movable member.

### [Description of Drawings]

FIG. 1 is a schematic view illustrating a moving device according to a first embodiment of the present invention.
FIG. 2 is a perspective view illustrating a portion of the moving device illustrated in FIG. 1.
FIG. 3 is a perspective view of a stationary member of the moving device illustrated in FIG. 1.
FIGS. 4 and 5 are perspective views of a first movable member of the moving device illustrated in FIG. 1 at different angles.
FIGS. 6 and 7 are views for describing an operating process of the moving device illustrated in FIG. 1.
FIGS. 8 and 9 are views illustrating a modified example of the stationary member and the first movable member illustrated in FIG. 1.
FIGS. 10 to 12 are schematic views illustrating a moving device according to a second embodiment of the present invention that are views for describing an operating process thereof.
FIGS. 13 to 15 are views illustrating a modified example of a holder and a driving unit illustrated in FIG. 10.
FIGS. 16 to 18 are schematic views illustrating a moving device according to a third embodiment of the present invention that are views for describing an operating process thereof.
FIGS. 19 to 22 are views illustrating a modified example of a holder and a driving unit illustrated in FIG. 16.
FIGS. 23 to 26 are schematic views illustrating a moving device according to a fourth embodiment not part of the present invention that are views for describing an operating process thereof.
FIGS. 27 and 28 are schematic views illustrating a moving device according to a fifth embodiment not part of the present invention.
FIG. 29 is an exploded perspective view of the moving device illustrated in FIG. 27.
FIGS. 30 to 32 are views for describing a process in which a first movable member of the moving device illustrated in FIG. 27 relatively moves with respect to a stationary member and a second movable member.
FIGS. 33 to 35 are views for describing a process in which the first movable member and the second movable member of the moving device illustrated in FIG. 27 relatively move with respect to the stationary member.
FIG. 36 is a view for describing one example of a method of operating the first movable member illustrated in FIG. 27.
FIG. 37 is a cross-sectional view of the stationary member, the first movable member, and the second movable member illustrated in FIG. 27.

### [Modes of the Invention]

Hereinafter, embodiments of the present invention will be described in detail with reference to the accompanying drawings so that those skilled in the art to which the present invention pertains may easily carry out the present invention. The present invention may be implemented in various different forms and is not limited to the embodiments described herein. In order to clearly describe the present invention, parts not related to the description have been omitted in the accompanying drawings, and identical or similar components are denoted by the same reference numerals throughout the specification.

In the specification, it should be understood that the term "include" or "have" is intended to describe that a feature, a number, a step, an operation, a component, a part, or a combination thereof described in the specification is present, but does not preclude the possibility of the presence or addition of one or more other features, numbers, steps, operations, components, parts, or combinations thereof.

When a first component is described as being "in front of," "behind," "above," or "under" a second component, it includes not only a case in which the first component is disposed "in front of," "behind," "above," or "under" the second component in direct contact therewith, but also a case in which a third component is disposed therebetween unless there are special circumstances. In addition, when a first component is described as being "connected" to a second component, it includes not only a case in which the first component and the second component are directly connected, but also a case in which the first component and the second component are indirectly connected unless there are special circumstances.

A moving device according to one embodiment of the present invention is a device for causing relative movement between a stationary member and a first movable member provided at one side of a tubular member, and the present invention relates to a moving device for causing relative movement between the first movable member and the stationary member to which one side of a tendon or sheath is coupled by causing relative movement between the tendon, which is a thin and linear member with bendability and a predetermined tensile strength, and the sheath, which is a tubular member through which the tendon passes and which has bendability and a predetermined compressive strength.

In this case, the tubular member may be a member formed to extend in a longitudinal direction and having bendability and flexibility which may be bent in one direction, and the stationary member and the first movable member may be portions of a device installed at one side of the tubular member to perform a predetermined function.

In the present embodiment, when the tendon is inserted into the sheath, it means that one end portion or a portion of the tendon enters an opening provided in one end portion of the sheath, when the tendon is drawn out from the sheath, it means that the one end portion or portion of the tendon accommodated inside the sheath is drawn out from the opening provided in the one end portion of the sheath, and when the driving unit releases the tendon or sheath, it means that the driving unit slightly releases the restraint of the tendon or sheath to smooth the relative movement between the tendon and the sheath.

For example, although the tubular member may be configured as an endoscope and the stationary member and the first movable member may be configured as portions of an end effector provided on an end portion of the endoscope to perform a predetermined function, for example, surgery or surgical procedure using the endoscope, the tubular member, the stationary member, and the first movable member are not limited thereto.

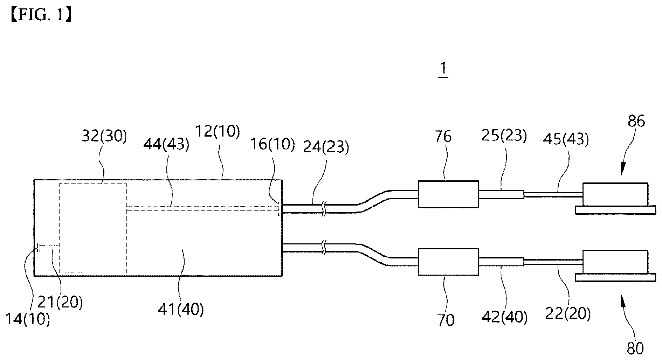

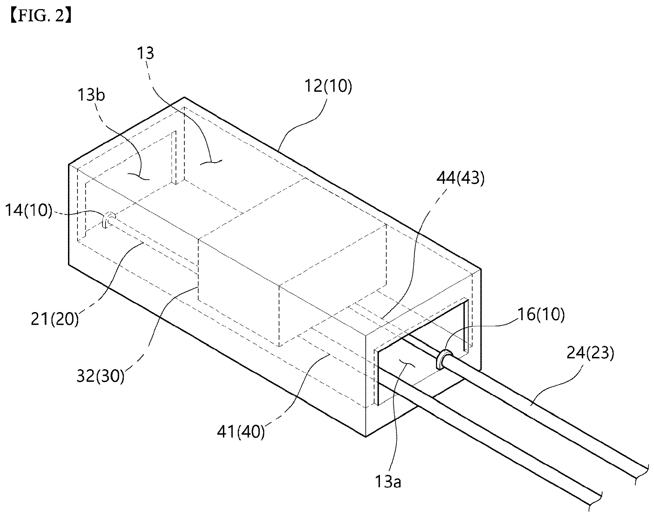

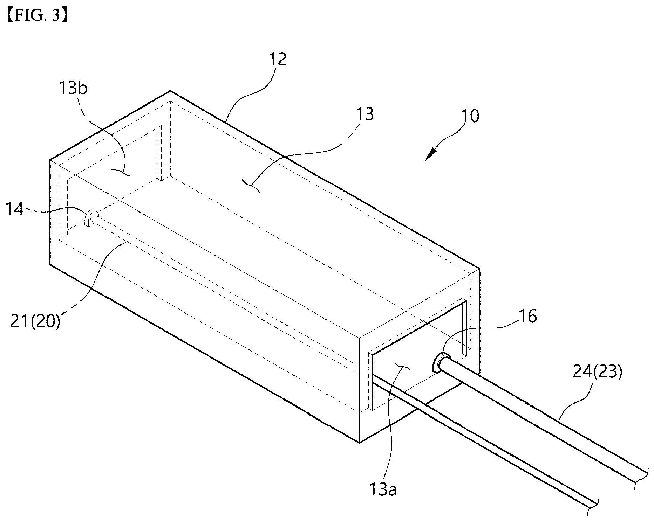

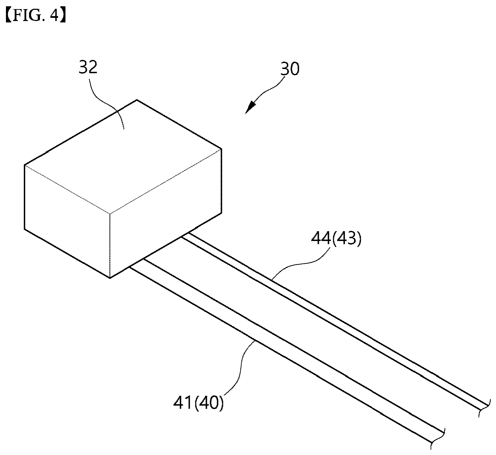

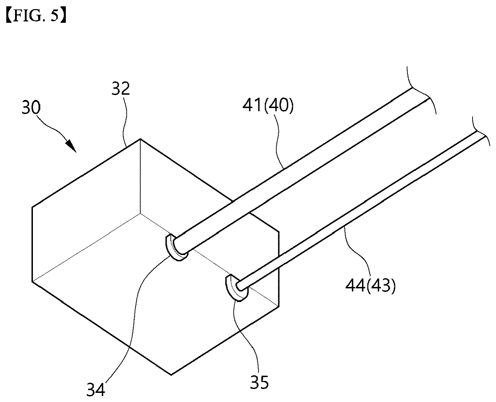

FIG. 1 is a schematic view illustrating a moving device according to a first embodiment of the present invention. FIG. 2 is a perspective view illustrating a portion of the moving device illustrated in FIG. 1. FIG. 3 is a perspective view of a stationary member of the moving device illustrated in FIG. 1. FIGS. 4 and 5 are perspective views of a first movable member of the moving device illustrated in FIG. 1 at different angles.

Hereinafter, in describing FIGS. 1 to 9, the description will be made by defining a left direction of FIG. 1 as a forward direction and a right direction as a rearward direction. Meanwhile, in FIGS. 2 and 3, components projected inside a stationary member are marked by dotted lines.

Referring to FIG. 1, a moving device 1 according to the first embodiment of the present invention may include a stationary member 10, a first tendon 20, a second sheath 23, a first movable member 30, a first sheath 40, a second tendon 43, first and second holders 70 and 76, and first and second driving units 80 and 86.

Referring to FIGS. 2 and 3, the stationary member 10 may be configured as a body 12 with a rectangular parallelepiped shape, and openings 13a and 13b may be respectively formed at one side and the other side, for example, front and rear sides of the body 12.

In this case, a shape of the body 12 of the stationary member 10 may be modified or changed variously such as being formed to be curved to include a curved surface to fit the shape of a tubular member to which the stationary member 10 is coupled.

A guide hole 13 formed to extend in a predetermined direction and accommodating the first movable member 30 is formed inside the body 12. The first movable member 30 is coupled to relatively move in an extending direction of the guide hole 13 with respect to the stationary member 10 inside the guide hole 13.

In this case, although the guide hole 13 is formed linearly in a longitudinal direction of the body 12 in the present embodiment, in another embodiment, the extending direction of the guide hole 13 may be formed along a curve. For example, the guide hole 13 may be formed to have one curved section to be suitable for a function performed by the first movable member 30 or function performed by a predetermined member installed at one side of the first movable member 30.

In addition, although the first movable member 30 is accommodated inside the stationary member 10 to be protected from the outside in the present embodiment, in another embodiment, the guide hole 13 may be modified to a guide groove formed in an outer side portion of the stationary member 10, and the first movable member 30 may be formed to move relatively in an extending direction of the guide groove outside the stationary member 10.

A first tendon 20 made of a flexible material and having a thin and long shape is coupled to one side, for example, a front end portion of the stationary member 10. To this end, a first tendon fixing part 14 to which one side 21 of the first tendon 20 is fixed is formed on the front end portion of the stationary member 10. The first tendon fixing part 14 may be configured as a coupling hole to which the first tendon 20 is insertion-coupled. The first tendon 20 is disposed to extend rearward.

The second sheath 23 with a hollow shape capable of accommodating the tendon therein is coupled to the other side, for example, a rear end portion of the stationary member 10. To this end, a second sheath fixing part 16 to which one side 24 of the second sheath 23 is coupled is formed on the rear end portion of the stationary member 10. The second sheath fixing part 16 may be configured as a coupling hole to which the second sheath 23 is insertion-coupled.

In this case, as long as the first tendon 20 and the second sheath 23 may be coupled, structures of the first tendon fixing part 14 and the second sheath fixing part 16 are not particularly limited. For example, the first tendon 20 may be fixed to the stationary member 10 by welding, an adhesive, or the like without a separate fixing part.

Referring to FIGS. 2, 4, and 5, the first movable member 30 may be configured as a body 32 disposed in the guide hole 13. The first sheath 40 with a hollow shape capable of accommodating the tendon therein and the second tendon 43 made of a flexible material and having a thin and long shape are coupled to the first movable member 30.

To this end, a first sheath fixing part 34 to which one side 41 of the first sheath 40 is coupled and a second tendon fixing part 35 to which one side 44 of the second tendon 43 are coupled are formed on the first movable member 30. In this case, as described above in relation to the first tendon fixing part 14 and the second sheath fixing part 16, structures of the first sheath fixing part 34 and the second tendon fixing part 35 are not particularly limited as long as the first sheath 40 and the second tendon 43 may be fixed thereto.

The first sheath 40 allows the first tendon 20 coupled to the stationary member 10 to pass therethrough and extends rearward, and the second tendon 43 passes through the second sheath 23 coupled to the stationary member 10 and extends rearward.

Meanwhile, in order to prevent the first movable member 30 from vibrating or rotating in a direction perpendicular to the extending direction when the first movable member 30 moves in the extending direction of the guide hole 13, a cross section of the first movable member 30 in a longitudinal direction may have a shape corresponding to a cross section of the guide hole 13.

In this case, in order to prevent the vibrations or rotation of the first movable member 30 and guide the movement of the first movable member 30, a guide protrusion (not illustrated) and a guide groove (not illustrated) may be provided on the first movable member 30 and an inner wall of the guide hole 13 of the stationary member 10.

Meanwhile, the first movable member 30 may be formed to relatively move with respect to the stationary member 10 between the first tendon fixing part 14 to which one side of the first tendon 20 is coupled and the second sheath fixing part 16 to which the one side 24 of the second sheath 23 is coupled.

In other words, the first tendon fixing part 14 and the second sheath fixing part 16 may become a reference for determining a movement section of the first movable member 30. Therefore, by adjusting relative positions between the first tendon fixing part 14 and the second sheath fixing part 16, a length and path of the movement section of the first movable member 30 may be set.

Meanwhile, in order to prevent the first movable member 30 from being separated from a portion between the first tendon fixing part 14 and the second sheath fixing part 16 (or the guide hole 13) and exiting to the outside of the stationary member 10, the stationary member 10 may be provided with a stopper capable of restricting the relative movement section of the first movable member 30. For example, since the cross section of the first movable member 30 in the longitudinal direction may be formed to be larger than the openings 13a and 13b, edge portions of the openings 13a and 13b may serve as the stoppers.

In this case, referring back to FIG. 2, when the stationary member 10 moves rearward together with the first tendon 20 as the first tendon 20 is pulled rearward, the relative movement may occur between the stationary member 10 and the first movable member 30.

Alternatively, when the first movable member 30 is moved rearward by tension applied to the second tendon 43 as the second tendon 43 is pulled rearward, the relative movement may occur between the first movable member 30 and the stationary member 10.

In this case, in order to smoothly cause the relative movement, an angle between a relative movement direction between the stationary member 10 and the first movable member 30 and a direction of a force (i.e., tension of the tendon) applied to the stationary member 10 and the first movable member 30 is preferably as small as possible.

To this end, the first tendon 20 and the second tendon 43 may be disposed parallel to each other in the extending direction of the guide hole 13. **In** addition, the second sheath 23 and the first sheath 40 may extend rearward and may be disposed parallel to each other.

Referring back to FIG. 1, the other side 22 of the first tendon 20 may be coupled to the first driving unit 80 and pulled to one side, for example, rearward based on FIG. 1 by the first driving unit 80. Therefore, the other side 22 of the first tendon 20 may be drawn out from the other side 42 of the first sheath 40.

The other side 45 of the second tendon 43 may be coupled to the second driving unit 86 and pulled to one side, for example, rearward based on FIG. 1 by the second driving unit 86. Therefore, the other side 45 of the second tendon 43 may be drawn out from the other side 25 of the second sheath 23.

In this case, the first and second driving units 80 and 86 may have various configurations capable of pulling the first and second tendons 20 and 43. For example, the first and second driving units 80 and 86 may be configured as actuators or operating members which may be manipulated manually.

In other words, the first and second driving units 80 and 86 may be configured as motors for rotating pulleys around which the first and second tendons 20 and 43 are wound, respectively. Alternatively, the first and second driving units 80 and 86 may be configured as operating members to which the first and second tendons 20 and 43 are respectively coupled and which are moved by linear actuators.

The other side 25 of the second sheath 23 is fixed by the second holder 76. The second holder 76 serves to fix the other side 25 of the second sheath 23 to prevent the other side 25 of the second sheath 23 from being moved rearward by a friction force together with the second tendon 43 even when the second tendon 43 is pulled by the second driving unit 86. Therefore, the relative movement between the second sheath 23 and the second tendon 43 may occur.

Similarly, the other side 42 of the first sheath 40 is fixed by the first holder 70. The first holder 70 serves to fix the other side 42 of the first sheath 40 to prevent the other side 42 of the first sheath 40 from being moved rearward by a friction force together with the first tendon 20 even when the first tendon 20 is pulled by the first driving unit 80. Therefore, the relative movement between the first sheath 40 and the first tendon 20 may occur.

**In** this case, the first and second holders 70 and 76 may be configured as fixing frames to which the first and second sheaths 23 and 40 are insertion-coupled, respectively, but are not particularly limited thereto as long as the relative movement between the first and second holders 70 and 76 and the first and second tendons 20 and 43 may occur.

Hereinafter, the relative movement between the stationary member and the first movable member of the moving device according to the first embodiment of the present invention will be described in more detail.

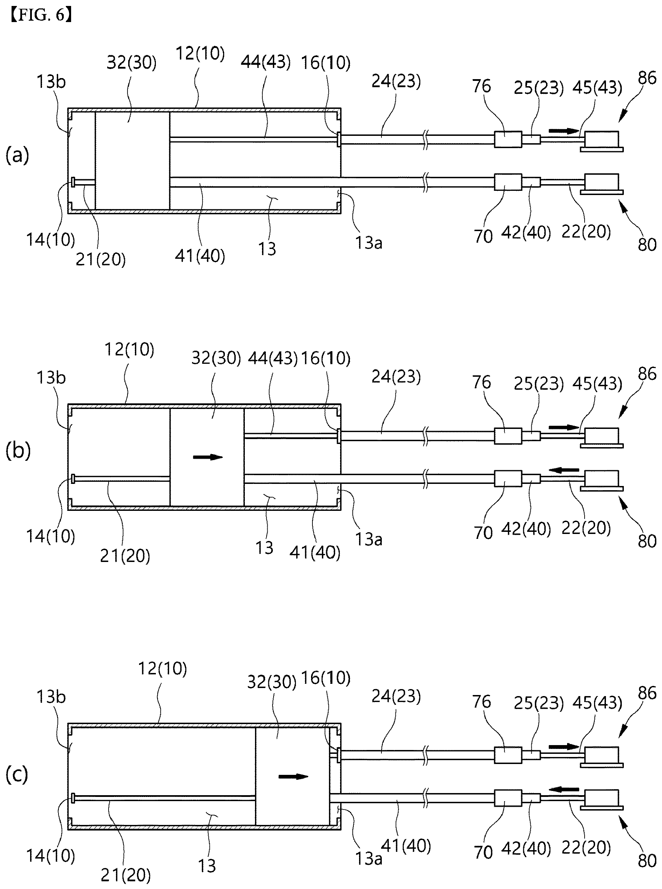

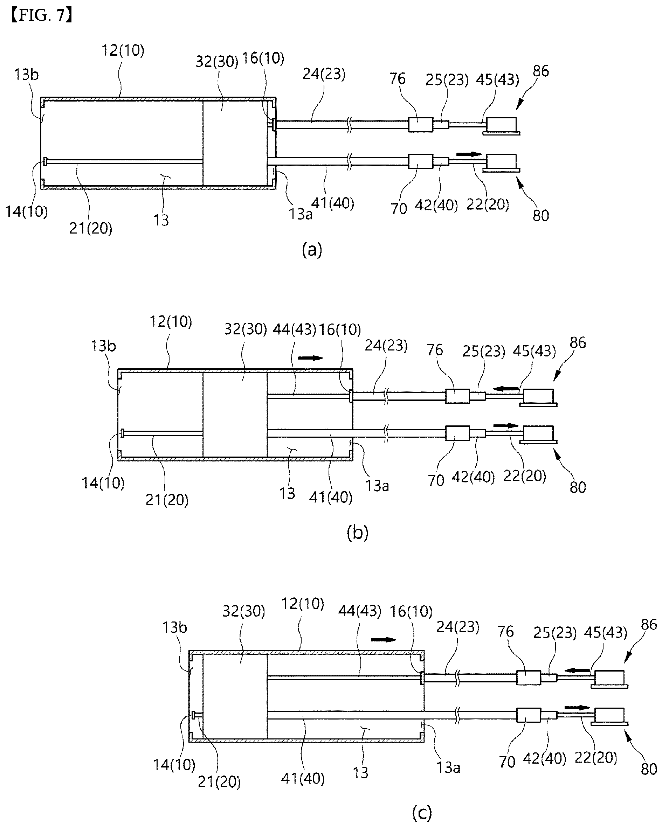

FIGS. 6 and 7 are views for describing an operating process of the moving device illustrated in FIG. 1.

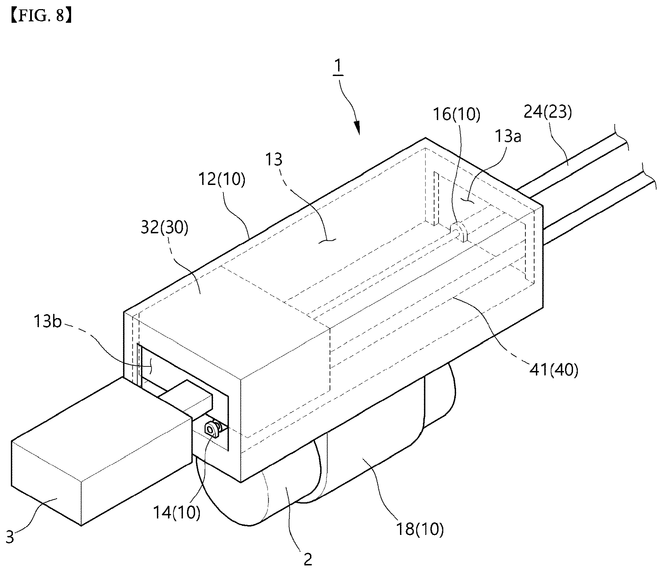

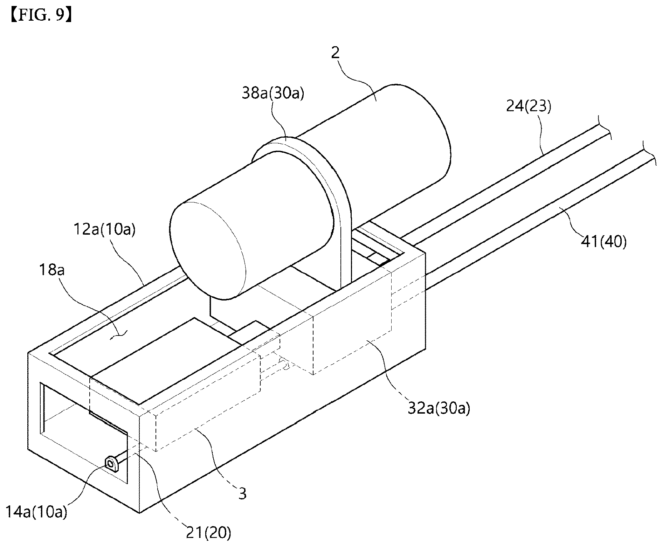

In FIGS. 6 and 7, a cross section of the stationary member is illustrated to allow the inside to be visible, and in FIGS. 8 and 9, components projected inside the stationary member are marked by dotted lines.

Referring to (a) of FIG. 6, the first movable member 30 is positioned at a front end portion side of the guide hole 13. In this case, a position of the first movable member 30 relative to the stationary member 10 is referred to as a first position. When the second driving unit 86 pulls the other side 45 of the second tendon 43 rearward, the other side 45 of the second tendon 43 is drawn out from the other side 25 of the second sheath 23, and the one side 44 of the second tendon 43 is inserted into the one side 24 of the second sheath 23, thereby causing the relative movement between the second tendon 43 and the second sheath 23.

Referring to (b) and (c) FIG. 6, as tension is applied to the second tendon 43, a force is applied to the first movable member 30 rearward, and the first movable member 30 relatively moves rearward with respect to the stationary member 10.

In this case, the first driving unit 80 releases the other side 22 of the first tendon 20 and provides a predetermined degree of freedom to the first tendon 20. Therefore, since the relative movement may occur between the first tendon 20 and the first sheath 40 such that the other side 22 of the first tendon 20 is slightly inserted into the first sheath 40, the first movable member 30 may be smoothly moved rearward by the first tendon 20 or the first sheath 40 without resistance.

In this case, the first movable member 30 may move relative to the stationary member 10 until it is positioned at a rear end portion side of the guide hole 13. In this case, a position of the first movable member 30 relative to the stationary member 10 is referred to as a second position.

Referring to (a) of FIG. 7, the first movable member 30 may be positioned at the second position. When the first driving unit 80 pulls the other side 22 of the first tendon 20 rearward, the other side 22 of the first tendon 20 is drawn out from the other side 42 of the first sheath 40, and the one side 21 of the first tendon 20 is inserted into the one side 41 of the first sheath 40, thereby causing the relative movement between the first tendon 20 and the first sheath 40.

Referring to (b) and (c) of FIG. 7, as tension is applied to the first tendon 20, a force is applied to the stationary member 10 rearward, and the stationary member 10 relatively moves rearward with respect to the first movable member 30.

In this case, the second driving unit 86 releases the other side 45 of the second tendon 43 and provides a predetermined degree of freedom to the second tendon 43. Therefore, since the relative movement may occur between the second tendon 43 and the second sheath 23 such that the other side 45 of the second tendon 43 is slightly inserted into the second sheath 23, the stationary member 10 may be smoothly moved rearward by the second tendon 43 or the second sheath 23 without resistance.

Meanwhile, when the above-described relative movement is described based on the first movable member 30, the first movable member 30 relatively moves forward with respect to the stationary member 10. As described above, according to the present embodiment, the relative linear movement between the first movable member 30 and the stationary member 10 may occur in the extending direction of the guide hole 13.

Hereinafter, a modified example of the stationary member and the first movable member of the moving device according to the first embodiment of the present invention will be described. FIGS. 8 and 9 are views illustrating a modified example of the stationary member and the first movable member illustrated in FIG. 1.

Referring to FIG. 8, according to a modified example of the present embodiment, an endoscope fixing part 18 to which a front end portion 2 of an endoscope device may be coupled may be formed on an outer side portion of the stationary member 10. Therefore, the stationary member 10 may be fixedly positioned on the front end portion 2 of the endoscope device.

Meanwhile, a surgical member 3 for an endoscope device may be coupled to the front side of the first movable member 30. For example, the surgical member 3 for an endoscope device may be configured as a surgical forceps or scalpel.

**In** this case, the front opening 13b of the stationary member 10 may be formed to have a size sufficient for the surgical member 3 for an endoscope device to pass through the front opening 13b.

Therefore, in a state in which the first movable member 30 is positioned at the second position, the surgical member 3 for an endoscope device may be positioned inside the guide hole 13 of the stationary member 10 and protected from the outside. In addition, when the stationary member 10 and the first movable member 30 reach target positions, the relative movement occurs so that the first movable member 30 is positioned at the first position, and thus the surgical member 3 for an endoscope device may protrude to the outside of the stationary member 10 and perform a predetermined function.

In addition, since the surgical member 3 for an endoscope device is accommodated inside the stationary member 10, it is possible to prevent damage to internal tissues of the human body which may be caused by the surgical member 3 for an endoscope device in a process of moving the stationary member 10 and the first movable member 30 to the target positions.

Referring to FIG. 9, according to another modified example of the present embodiment, a side open hole 18a connected to the guide hole is formed in a side portion of a stationary member 10a. In this case, the side open hole 18a may be formed to have a sufficient length in the extending direction of the guide hole corresponding to a relative movement distance between a first movable member 30a and the stationary member 10a.

An endoscope fixing part 38a passing through the side open hole 18a of the stationary member 10a and protruding outward is formed on a side portion of a body 32a of the first movable member 30a. The front end portion 2 of the endoscope device is coupled to the endoscope fixing part 38a. Therefore, the first movable member 30a may be fixedly positioned on the front end portion 2 of the endoscope device.

The surgical member 3 for an endoscope device is coupled to a front side of the body 32a of the first movable member 30. Therefore, the surgical member 3 for an endoscope device and the front end portion 2 of the endoscope device may be moved integrally with the first movable member 30a.

Therefore, the front end portion 2 of the endoscope device and the surgical member 3 for an endoscope device may not relatively move with respect to each other. In addition, the first movable member 30a may be positioned at the second position so that the surgical member 3 for an endoscope device can be protected from the outside by the stationary member 10a, and the first movable member 30a may be positioned at the first position so that the surgical member 3 for an endoscope device may be exposed to the outside of the stationary member 10a.

Hereinafter, a moving device according to a second embodiment of the present invention will be described.

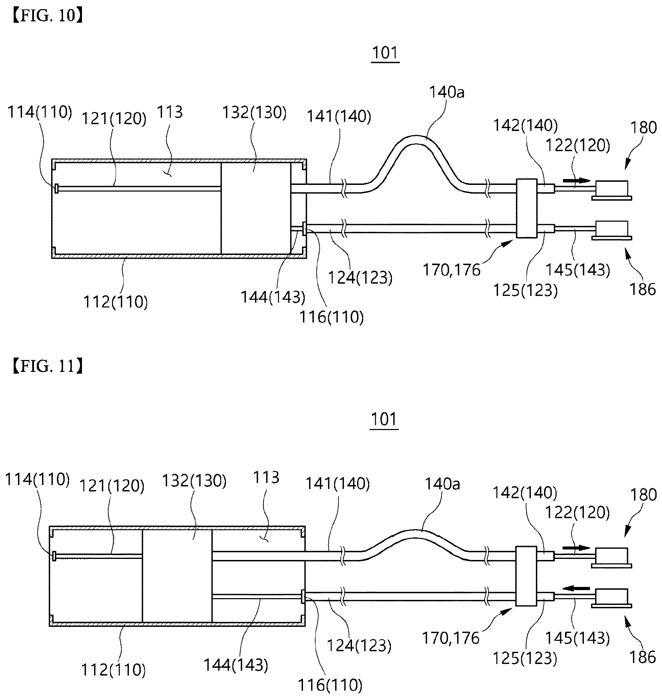

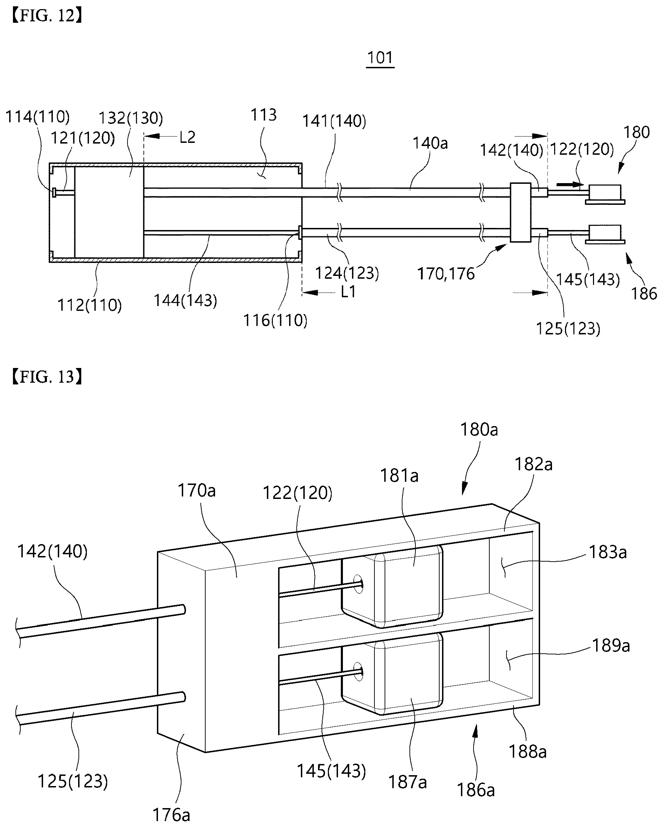

FIGS. 10 to 12 are schematic views illustrating a moving device according to a second embodiment of the present invention that are views for describing an operating process thereof. FIGS. 13 to 15 are views illustrating a modified example of a holder and a driving unit illustrated in FIG. 10.

Hereinafter, in describing FIGS. 10 to 15, the description will be made by defining a left direction of FIG. 10 as a forward direction and a right direction as a rearward direction. Meanwhile, in FIGS. 10 to 13, a cross section of the stationary member is illustrated to allow the inside to be visible.

Referring to FIG. 10, a moving device 101 according to a second embodiment of the present invention may include a stationary member 110, a first tendon 120, a second sheath 123, a first movable member 130, a first sheath 140, a second tendon 143, first and second holders 170 and 176, and first and second driving units 180 and 186.

In this case, in the moving device 101 according to the second embodiment, since the stationary member 110, the first tendon 120, the first movable member 130, the second tendon 143, the first and second holders 170 and 176, and the first and second driving units 180 and 186 may have the same configurations as those of the first embodiment of the present invention, detailed descriptions thereof will be omitted.

In other words, the first movable member 130 may be coupled to move relative to a guide hole 113 of the stationary member 110 and may move between first and second positions along the guide hole 113, one side 144 of the second tendon 143 is coupled to the first movable member 130, and a first tendon fixing part 114 to which one side 121 of the first tendon 120 is coupled is formed on a front end portion of the stationary member 110. The other side 122 of the first tendon 120 is coupled to the first driving unit 180 to be pulled by the first driving unit 180, and the other side 145 of the second tendon 143 is coupled to the second driving unit 186 to be pulled by the second driving unit 186. When the other side 142 of the first sheath 140 to be described below is fixedly coupled to the first holder 170 and the first tendon 120 is pulled by the first driving unit 180, the other side 122 of the first tendon 120 is drawn out from the first sheath 140, and relative movement between the first tendon 120 and the first sheath 140 occurs. When the other side 125 of the second sheath 123 to be described below is fixedly coupled to the second holder 186 and the second tendon 143 is pulled by the second driving unit 186, the other side 145 of the second tendon 143 is drawn out from the second sheath 123, and relative movement between the second tendon 143 and the second sheath 123 occurs.

Hereinafter, an operating process of the second sheath 123, the first sheath 140, and the moving device 101 according to the present embodiment will be described in more detail.

Referring to FIGS. 10 to 12, one side 141 of the first sheath 140 is coupled to the first movable member 130, and one side 124 of the second sheath 123 is coupled to a rear end portion of the stationary member 110. In this case, in a state in which the first movable member 130 is positioned at the second position, the first sheath 140 may be disposed to be curved.

More specifically, the first sheath 140 may include a curved portion 140a formed to be curved outward in the state in which the first movable member 130 is positioned at the second position. Meanwhile, in FIG. 10, although it is illustrated that the curved portion 140a is formed intensively in one section of the first sheath 140, the curved portion 140a may be provided as a plurality of curved portions having slightly small sizes and distributed in a plurality of sections of the first sheath 140.

Referring to FIGS. 10 and 11, when the first driving unit 180 pulls the other side 122 of the first tendon 120 in the state in which the first movable member 130 is positioned at the second position, the other side 122 of the first tendon 120 is drawn out from the other side 142 of the first sheath 140, and the one side 121 of the first tendon 120 is inserted into the one side 141 of the first sheath 140, thereby causing the relative movement between the first tendon 120 and the first sheath 140.

When the other side 122 of the first tendon 120 is drawn out from the first sheath 140, an interaction force is generated between the first tendon 120 and the first sheath 140, and thus the first tendon 120 presses the first sheath 140 rearward, and this force (compressive force) is intensively applied to the curved portion 140a.

At this time, a repulsive force acts forward on the curved portion 140a of the first sheath 140 by the compressive force applied to the first sheath 140 and a supporting force of the first holder 170, and the curved portion 140a is straightened due to a curvature reduced by the repulsive force.

Therefore, the one side 141 of the first sheath 140 pushes the first movable member 130 forward, and the first movable member 130 linearly relatively moves forward with respect to the stationary member 110.

Meanwhile, the second driving unit 186 releases the other side 145 of the second tendon 143 and provides a predetermined degree of freedom to the second tendon 143. Therefore, since the relative movement may occur between the second tendon 143 and the second sheath 123 such that the other side 145 of the second tendon 143 is slightly inserted into the second sheath 123, the first movable member 130 may be smoothly moved forward by the second tendon 143 without resistance.

Referring to FIG. 12, when the first sheath 140 and the second sheath 123 are disposed parallel to each other as the curved portion 140a of the first sheath 140 is straightened, the first movable member 130 may be positioned at the first position. In this case, in order to more quickly move or completely move the first movable member 130 to the first position, it is preferable that the first sheath 140 be configured to continuously apply the repulsive force to the first movable member 130 forward even in the state in which the first movable member 130 is positioned at the first position.

In other words, it is preferable that the first sheath 140 be disposed to be slightly curved even in the state in which the first movable member 130 is positioned at the first position and configured so that the curved portion 140a of the first sheath 140 remains.

Meanwhile, when the second driving unit 186 pulls the other side 145 of the second tendon 143 and the first driving unit 180 releases the other side 122 of the first tendon 120, the relative movement between the second tendon 143 and the second sheath 123 occurs, and the first movable member 130 is relatively moved rearward with respect to the stationary member 110 by the tension applied to the second tendon 143.

At the same time, the other side 122 of the first tendon 120 is slightly inserted into the first sheath 140, and as the first movable member 130 moves rearward, the curved portion 140a is re-formed in a predetermined section of the first sheath 140.

As described above, according to the present embodiment, the relative linear movement between the first movable member 130 and the stationary member 110 may occur using the compressive force and repulsive force applied to the first sheath 140.

Hereinafter, a modified example of the first and second driving units of the moving device according to the second embodiment of the present invention will be described.

Referring to FIG. 13, first and second holders 170a and 176a and first and second driving units 180a and 186a of the moving device according to the modified example of the present embodiment may be formed integrally. More specifically, the first and second holders 170a and 176a and the first and second driving units 180a and 186a may include a first body part 182a positioned at one side, for example, an upper side based on FIG. 13 and a second body part 188a positioned under the first body part 182a.

A first guide groove 183a which is formed in the extending direction of the first tendon 120 and of which one side is open is formed inside the first body part 182a, and a first operating member 181a is coupled to move in the extending direction inside the first guide groove 183a. The other side 122 of the first tendon 120 is fixedly coupled to the first operating member 181a. The first holder 170a to which the other side 142 of the first sheath 140 is fixed is formed at a front side of the first body part 182a.

A second guide groove 189a which is formed in the extending direction of the second tendon 143 and of which one side is open is formed inside the second body part 188a, and a second operating member 187a is coupled to move in the extending direction inside the second guide groove 189a. The other side 145 of the second tendon 143 is fixedly coupled to the second operating member 187a. The second holder 176a to which the other side 125 of the second sheath 123 is fixed is formed at a front side of the second body part 188a.

Therefore, a user may move any one of the first operating member 181a and the second operating member 187a rearward and release the other one, thereby causing the relative movement between the first movable member and the stationary member.

For example, by moving the first operating member 181a rearward to pull the first tendon 120 rearward and releasing the restraint of the second operating member 187a to release the second tendon 143, the first movable member may be relatively moved forward with respect to the stationary member.

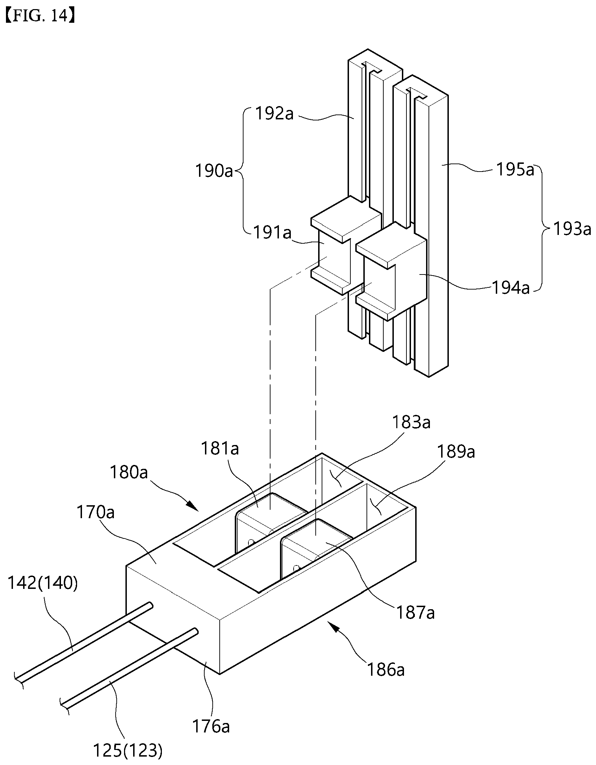

Referring to FIG. 14, the first and second driving units 180a and 186a of the moving device according to another modified example of the present embodiment may further include a first actuator 190a and a second actuator 193a. In this case, since components of the first and second driving units 180a and 186a except for the first and second actuators 190a and 193a may be the same as those of the above-described modified example, descriptions thereof will be omitted.

The first actuator 190a may include a first coupling member 191a coupled to the first operating member 181a and a first power generator 192a for generating power for moving the first coupling member 191a forward and rearward.

Similar to the first actuator 190a, the second actuator 193a may include a second coupling member 194a coupled to the second operating member 187a and a second power generator 195a for generating power for moving the second coupling member 194a forward and rearward.

According to the present modified example, since the user may move any one of the first operating member 181a and the second operating member 187a rearward and release the other one by controlling the operation of the first and second actuators 190a and 193a, the relative movement between the first movable member and the stationary member may occur.

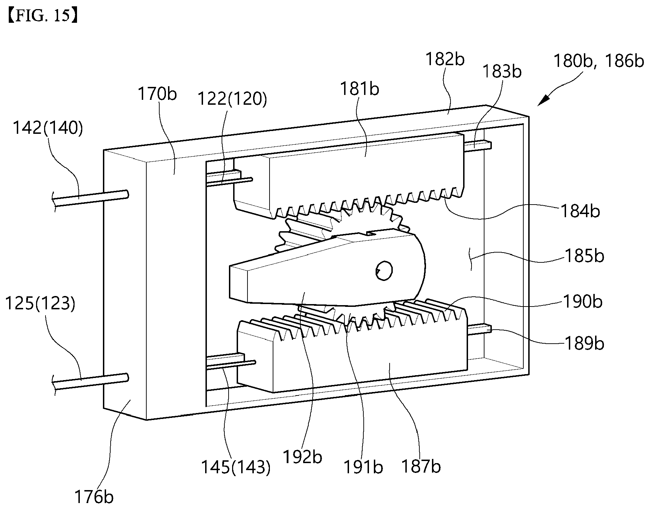

Referring to FIG. 15, first and second holders 170b and 176b and first and second driving units 180b and 186b of the moving device according to still another modified example of the present embodiment may be formed integrally. More specifically, the first and second holders 170b and 176b and the first and second driving units 180b and 186b may be configured as a body part 182b formed to extend in longitudinal directions of the first and second tendons 120 and 143.

A guide groove 185b of which one side is open and which is formed in the extending direction is formed inside the body part 182b. In this case, the guide groove 185b is formed to have a sufficient width in an arrangement direction of the first and second tendons 120 and 143 so that the first and second tendons 120 and 143 may pass the inside of the guide groove 185b in parallel.

The first and second holders 170b and 176b to which the first and second sheaths 123 and 140 are respectively coupled are formed at a front side of the body part 182b.

A pair of first and second guide protrusions 183b and 189b formed in an extending direction of the guide groove 185b and disposed to be spaced a predetermined distance from each other in a direction perpendicular to the extending direction are formed on an inner wall of the guide groove 185b.

A first operating member 181b in which a groove is formed to correspond to the first guide protrusion 183b is coupled to the first guide protrusion 183b to move in an extending direction of the first guide protrusion 183b.

A second operating member 187b in which a guide groove is formed to correspond to the second guide protrusion 189b is coupled to the second guide protrusion 189b to move in an extending direction of the second guide protrusion 189b. The other side 122 of the first tendon 120 is fixedly coupled to the first operating member 181b, and the other side 145 of the second tendon 143 is fixedly coupled to the second operating member 187b.

**In** this case, a first gear member 184b with a rack gear shape is formed on the first operating member 181b in a direction parallel to the extending direction of the guide groove 185b, and a second gear member 190b with a rack gear shape is also formed on the second operating member 187b in the parallel direction. In this case, the first gear member 184b and the second gear member 190b are disposed to face each other.

A third gear member 191b simultaneously circumscribing the first gear member 184b and the second gear member 190b is disposed between the first operating member 181b and the second operating member 187b. An operating member 192b to which an external force for rotating the third gear member 191b in one direction may be applied is provided at one side of the third gear member 191b.

When the user rotates the third gear member 191b in one direction by applying the external force to the operating member 192b, any one of the first and second operating members 181b and 187b moves rearward and the other one moves forward, and any one of the first and second tendons 120 and 143 is pulled rearward and the other one is released forward.

Therefore, according to the present modified example, the user may conveniently cause the relative movement between the first movable member and the stationary member by manipulating only the operating member 191b.

Of course, since the operating member 192b is provided with a power transmission member capable of receiving power from the actuator, the user may control the actuator to cause the relative movement between the first movable member and the stationary member.

Hereinafter a moving device according to a third embodiment of the present invention will be described.

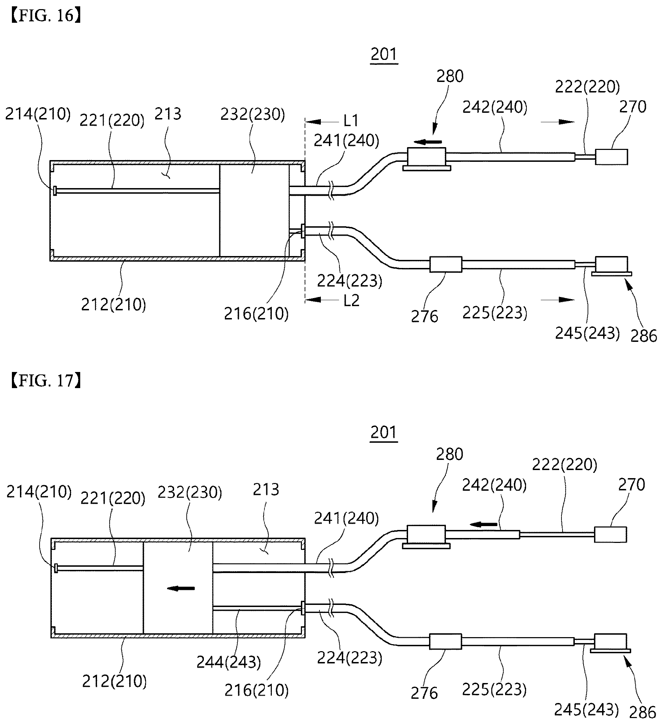

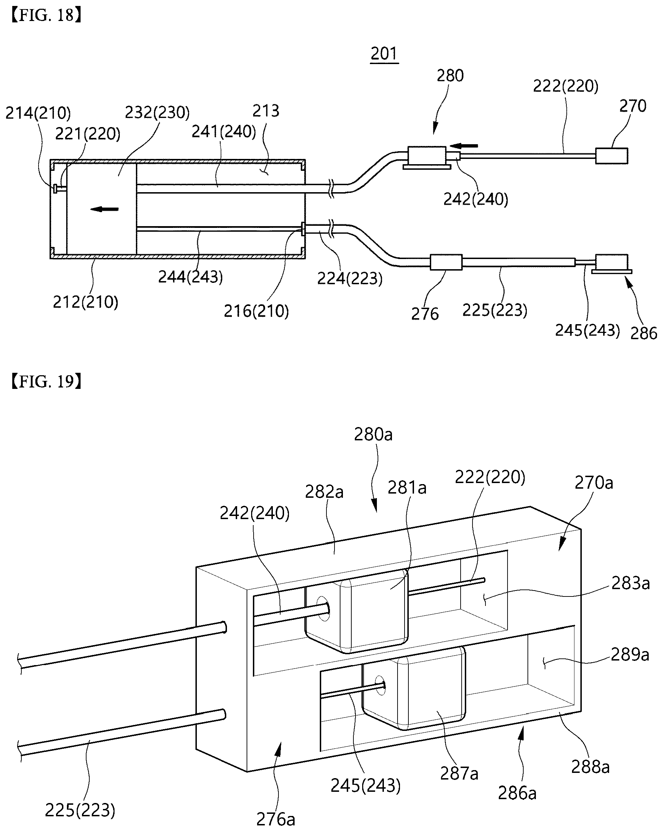

FIGS. 16 to 18 are schematic views illustrating a moving device according to a third embodiment of the present invention that are views for describing an operating process thereof. FIGS. 19 to 22 are views illustrating a modified example of a holder and a driving unit illustrated in FIG. 16.

Hereinafter, in describing FIGS. 16 to 22, the description will be made by defining a left direction of FIG. 16 as a forward direction and a right direction as a rearward direction. Meanwhile, in FIGS. 16 to 18, a cross section of the stationary member is illustrated to allow the inside to be visible.

Referring to FIG. 16, a moving device 201 according to a third embodiment of the present invention may include a stationary member 210, a first tendon 220, a second sheath 223, a first movable member 230, a first sheath 240, a second tendon 243, first and second holders 270 and 276, and first and second driving units 280 and 286.

In this case, in the moving device 201 according to the third embodiment, since the stationary member 210, the first tendon 220, the second sheath 223, the first movable member 230, the first sheath 240, the second tendon 243, the second holder 276, and the second driving unit 286 may have the same configurations as those of the first embodiment of the present invention, detailed descriptions thereof will be omitted.

In other words, the first movable member 230 may be coupled to move relative to a guide hole 213 of the stationary member 210 and may move between first and second positions along the guide hole 213, one side 244 of the second tendon 243 is coupled to the first movable member 230, and a first tendon fixing part 214 to which one side 221 of the first tendon 220 is coupled is formed on a front end portion of the stationary member 210. The other side 245 of the second tendon 243 is coupled to the second driving unit 286 to be pulled by the second driving unit 286. When the other side 225 of the second sheath 223 to be described below is fixedly coupled to the second holder 286 and the second tendon 243 is pulled by the second driving unit 286, the other side 245 of the second tendon 243 is drawn out from the second sheath 223, and relative movement between the second tendon 243 and the second sheath 223 occurs.

Hereinafter, an operating process of the first holder 270, the first driving unit 280, and the moving device 201 according to the present embodiment will be described in more detail.

In the present embodiment, the first holder 270 is provided to fix the other side 222 of the first tendon 220, and the first driving unit 280 is coupled adjacent to the other side 242 of the first sheath 240 and provided to move the first sheath 240. To this end, the first holder 270 is positioned relatively rearward, and the first driving unit 280 is positioned relatively forward.

Therefore, when the first driving unit 280 moves the first sheath 240 forward, the other side 222 of the first tendon 220 fixed by the first holder 270 is drawn out from the other side 242 of the first sheath 240, and the one side 221 of the first tendon 220 is inserted into one side 241 of the first sheath 240.

Referring back to FIG. 16, when the first driving unit 280 moves the first sheath 240 forward in the state in which the first movable member 230 is positioned at the second position, the one side 241 of the first sheath 240 is moved forward.

In this case, since the first sheath 240 has a predetermined compressive strength, the first sheath 140 may apply a force to the first movable member 230 forward as the first sheath 240 is moved forward by the first driving unit 280. Meanwhile, the second driving unit 286 releases the second tendon 243 and provides a predetermined degree of freedom.

Referring to FIGS. 17 and 18, the first movable member 230 to which a force is applied forward by the first sheath 240 relatively moves forward with respect to the stationary member 210 along the guide hole 213. In this case, as the first movable member 230 moves, the other side 245 of the second tendon 243 with the predetermined degree of freedom is inserted into the second sheath 223.

As described above, according to the present embodiment, the first driving unit 280 may directly move the first sheath 240 fixed to the first movable member 230 forward to relatively move the first movable member 230 forward with respect to the stationary member 210.

Meanwhile, when the second driving unit 286 pulls the other side 245 of the second tendon 243 rearward, the other side 245 of the second tendon 243 is drawn out from the other side 225 of the second sheath 223, and the one side 244 of the second tendon 243 is inserted into one side 224 of the second sheath 223. At the same time, the first driving unit 280 releases the other side 242 of the first sheath 240 and provides a predetermined degree of freedom to cause the relative movement between the first sheath 240 and the first tendon 220. Therefore, the first movable member 230 may relatively move rearward with respect to the stationary member 210.

As described above, according to the moving device 201 of the present embodiment, since the first driving unit 280 may directly move the first sheath 240 fixed to the first movable member 230, the relative movement occurs between the first movable member 230 and the stationary member 210 using the strength of the first sheath 240.

Meanwhile, in the present embodiment, the first holder 270, the second holder 276, the first driving unit 280, the second driving unit 286, and a difference between a length L1 of the second sheath 223 and a length L2 of the first sheath 240 are not particularly limited.

Hereinafter, a modified example of the third embodiment of the present invention will be described.

Referring to FIG. 19, first and second holders 270a and 276a and first and second driving units 280a and 286a of the moving device according to a modified example of the present embodiment may be formed integrally. More specifically, the first and second holders 270a and 276a and the first and second driving units 280a and 286a may include a first body part 282a positioned at one side, for example, an upper side based on FIG. 19 and a second body part 288a positioned under the first body part 282a.

A first guide groove 283a which is formed in the extending direction of the first tendon 220 and of which one side is open is formed inside the first body part 282a, and a first operating member 281a is coupled to move in the extending direction inside the first guide groove 283a. The other side 242 of the first sheath 240 is fixedly coupled to the first operating member 281a. The first holder 270a to which the other side 222 of the first tendon 220 is fixed is formed at a rear side of the first body part 182a.

A second guide groove 289a which is formed in the extending direction of the second tendon 243 and of which one side is open is formed inside the second body part 288a, and a second operating member 287a is coupled to move in the extending direction inside the second guide groove 289a. The other side 245 of the second tendon 243 is fixedly coupled to the second operating member 287a. The second holder 276a to which the other side 225 of the second sheath 223 is fixed is formed at a front side of the second body part 288a.

Therefore, a user moves the first operating member 281a forward or rearward and releases or moves the second operating member 187a in a direction parallel to a moving direction of the first operating member 281a, thereby causing the relative movement between the first movable member and the stationary member.

For example, by moving the first operating member 281a forward to move the first sheath 240 forward and releasing the restraint of the second operating member 287a to release the second tendon 143 or moving the second operating member 287a forward, the first movable member may be relatively moved forward with respect to the stationary member.

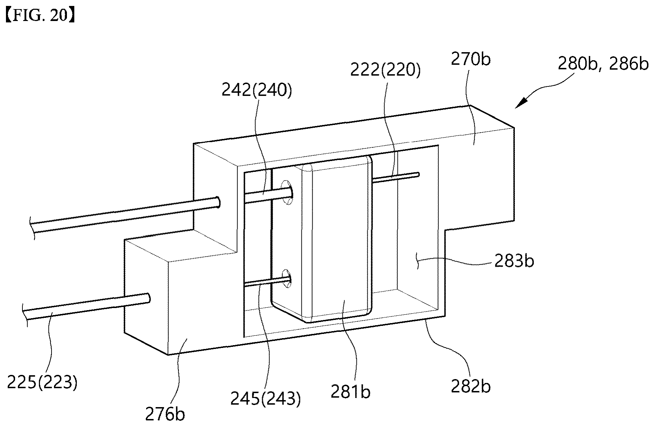

Referring to FIG. 20, in another modified example of the present embodiment, the first and second holders 270a and 276a, the first and second driving units 280a and 286a, and the above-described first and second operating members may be formed integrally.

More specifically, the first and second holders 270b and 276b and the first and second driving units 280b and 286b may be configured as a body part 282b formed to extend in longitudinal directions of the first and second tendons 220 and 243.

A guide groove 283b of which one side is open and which is formed in the extending direction is formed inside the body part 282b. In this case, the guide groove 283b is formed to have a sufficient width in an arrangement direction of the first and second tendons 220 and 243 so that the first and second tendons 220 and 243 may pass the inside of the guide groove 283b in parallel.

The first holder 270b to which the other side 222 of the first tendon 220 is fixedly coupled is formed at a rear side of the body part 282b, and the second holder 276b to which the other side 225 of the second sheath 223 is fixedly coupled is formed at a front side of the body part 282b.

An operating member 281b is coupled to the inside of the guide groove 283b to move in an extending direction of the guide groove 283b. In this case, the other side 242 of the first sheath 240 and the other side 245 of the second tendon 243 are fixedly coupled to the operating member 281b.

Therefore, according to the present modified example, the user may conveniently cause the relative movement between the first movable member and the stationary member by manipulating only one operating member 281b. For example, when the user moves the operating member 281b forward, the first sheath 240 pushes and moves the first movable member forward, and the second tendon 243 is released forward.

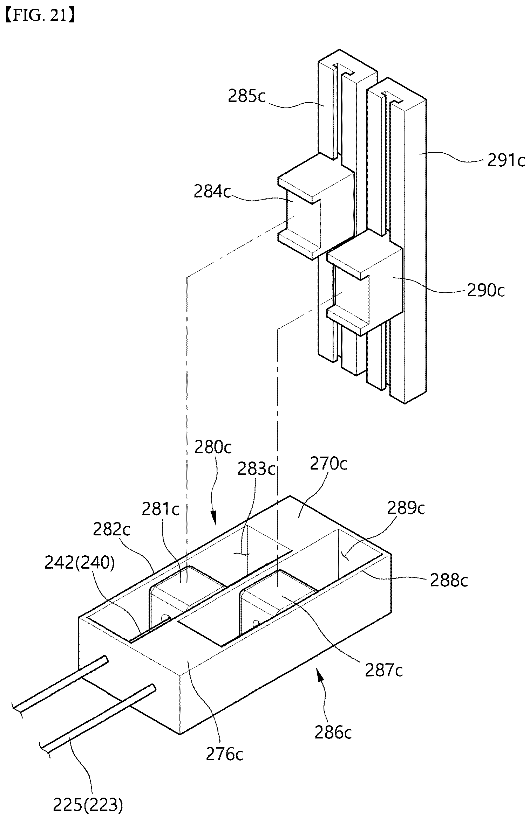

Referring to FIG. 21, in still another modified example of the present embodiment, the first and second driving units 280c and 286c may further include a first actuator 284c and 285c and a second actuator 290c and 291c.

In this case, since first and second operating members 281c and 287c, first and second body parts 282c and 288c, first and second guide grooves 283c and 289c, and first and second holders 270c and 276c in the present modified example may have the same configurations as those of the first and second operating members 281a and 287a (see FIG. 19), the first and second body parts 282a and 288a (see FIG. 19), and the first and second guide grooves 283a and 289a (see FIG. 19) in the modified example described above with reference to FIG. 19, descriptions thereof will be omitted.

In the present modified example, the first actuator 284c and 285c may include the first coupling member 284c coupled to the first operating member 281c and the first power generator 285c for generating power for moving the first coupling member 284c forward and rearward.

Similar to the first actuator 284c and 285c, the second actuator 290c and 291c may include the second coupling member 290c coupled to the second operating member 287c and the second power generator 291c for generating power for moving the second coupling member 290c forward and rearward.

According to the present modified example, since the user may control operations of the first and second actuators 284c, 285c, 290c, and 291c to move the first operating member 281c and release or move the second operating member 187a in a direction parallel to the first operating member 281c, the relative movement between the first movable member and the stationary member may occur.

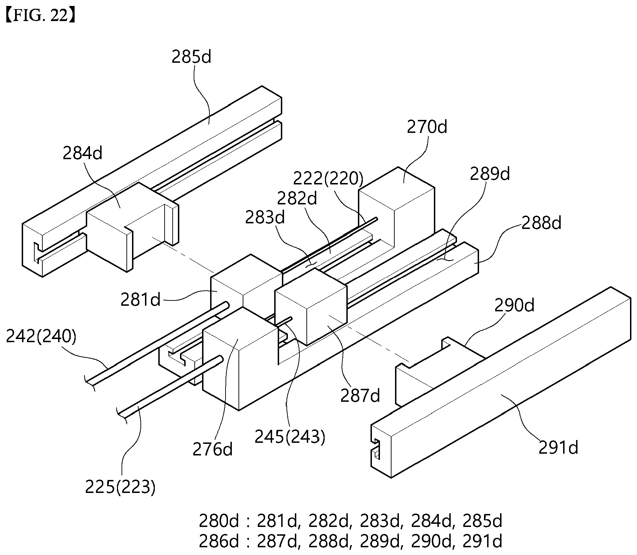

Referring to FIG. 22, in yet another modified example of the present embodiment, first and second holders 270d and 276d and first and second driving units 280d and 286d may be formed integrally. In this case, since first and second actuators 284d, 285d, 290d, and 291d of the present modified example may have the same configurations as those of the first and second actuators 284c, 285c, 290c, and 291c (see FIG. 21) of the modified example described above with reference to FIG. 21, descriptions thereof will be omitted.

In the present modified example, the first driving unit 280d includes a first body part 282d formed in a longitudinal direction of the first tendon 220. A first guide path 283d formed in the extending direction of the first tendon 220 is positioned at one side, for example, an upper side based on FIG. 22 of the first body part 282d.

The first operating member 281d is coupled to an upper side portion of the first body part 282d to move along the first guide path 283d. To this end, a guide protrusion and a guide groove may be formed on the first body part 282d and the first operating member 281d, respectively, along the first guide path 283d. The other side 242 of the first sheath 240 is fixedly coupled to the first operating member 281d.

Meanwhile, the second driving unit 286d includes a second body part 288d formed in a longitudinal direction of the second tendon 243. A second guide path 289d formed in the extending direction of the second tendon 243 is positioned at one side, for example, an upper side based on FIG. 22 of the second body part 288d.

The second operating member 287d is coupled to an upper side portion of the second body part 288d to move along the second guide path 289d. To this end, a guide protrusion and a guide groove may be formed on the second body part 288d and the second operating member 287d, respectively, along the second guide path 289d. The other side 245 of the second tendon 243 is fixedly coupled to the second operating member 287d.

The first holder 270d to which the other side 222 of the first tendon 220 is fixedly coupled is formed at a rear side of the first body part 282d, and the second holder 276d to which the other side 225 of the second sheath 223 is fixedly coupled is formed at a front side of the second body part 288d.

According to the present modified example, since the user may control operations of the first and second actuators 284d, 285d, 290d, and 291d to move the first operating member 281d and release or move the second operating member 187d in a direction parallel to the first operating member 281d, the relative movement between the first movable member and the stationary member may occur.

Hereinafter, a moving device according to a fourth embodiment of the present invention will be described.

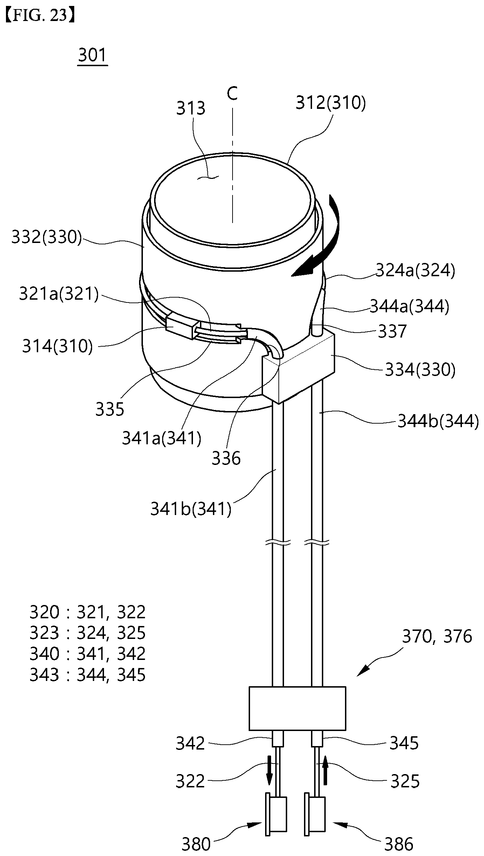

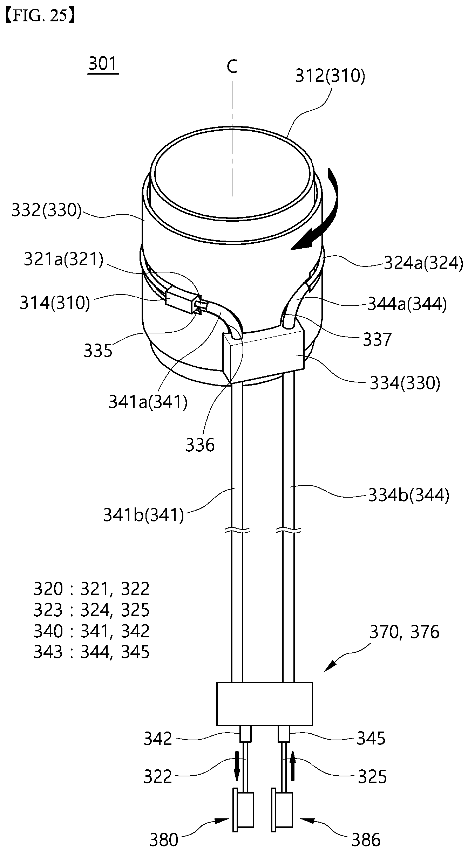

FIGS. 23 to 26 are schematic views illustrating a moving device according to a fourth embodiment not part of the present invention that are views for describing an operating process thereof.

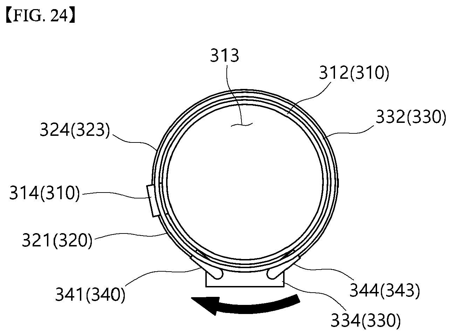

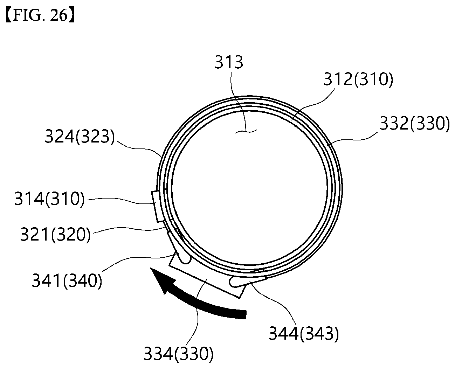

Hereinafter, in describing FIGS. 23 to 26, the description will be made by defining an upward direction of FIG. 23 as a forward direction, defining a downward direction as a rearward direction, and defining clockwise and counterclockwise based on FIG. 24. Meanwhile, in FIGS. 24 and 26, components other than the stationary member, the first movable member, the first and second tendons, and the first and second sheaths are omitted.

Referring to FIG. 23, a moving device 301 according to a third embodiment not part of the present invention may include a stationary member 310, first and second tendons 320 and 323, a first movable member 330, first and second sheaths 340 and 343, first and second holders 370 and 376, and first and second driving units 380 and 386.

In the present embodiment, a body part 312 of the stationary member 310 may be configured as a cylindrical member having a coupling hole 313 formed at the center thereof. A portion of the tubular member, for example, a front end portion (not illustrated) of an endoscope device may be fixedly coupled to the coupling hole 313.

A tendon fixing part 314 which protrudes outward and to which one side 321 of the first tendon 320 and one side 324 of the second tendon 323, which will be described below, are fixedly coupled is formed on an outer circumferential surface of the stationary member 310.

The body part 332 of the first movable member 330 is coupled to an outer portion of the stationary member 310 to rotate relative to each other. In this case, the body part 332 of the first movable member 330 may be configured as a cylindrical member and coaxially arranged with the stationary member 310 about a central axis C.

A sheath fixing part 334 which protrudes outward and to which one side 341 of the first sheath 340 and one side 344 of the second sheath 343, which will be described below, are coupled is formed on one side portion, for example, a lower side portion based on FIG. 23 of the body part 332 of the first movable member 330.

Meanwhile, a tendon fixing part guide hole 335 through which the tendon fixing part 314 of the stationary member 310 passes is formed in the body part 332 of the first movable member 330. In this case, the tendon fixing part guide hole 335 is formed in a circumferential direction of the body part 332.

Therefore, even when the first movable member 330 and the stationary member 310 rotate relative to each other about the central axis C, the tendon fixing part 314 is guided along the tendon fixing part guide hole 335, and thus the first movable member 330 may rotate relative to the stationary member 310 without being interfered by the tendon fixing part 314.

In this case, one end portion of the tendon fixing part guide hole 335 is connected to the first sheath guide hole 336 in which a first portion 341a of the first sheath 340 to be described below is disposed. In order to arrange the first sheath 340, one end portion, for example, a lower end portion based on FIG. 23 of the first sheath guide hole 336 is positioned adjacent to the sheath fixing part 334.

In this case, the first sheath guide hole 336 may be formed along a path curved at a predetermined curvature. More specifically, a portion in the first sheath guide hole 336 and the tendon fixing part guide hole 335 are connected may be formed in the circumferential direction of the body part 332, the lower end portion of the first sheath guide hole 336 may be formed in a longitudinal direction of the body part 332, and a portion positioned therebetween may be formed to be curved at a predetermined curvature.

The other end portion of the tendon fixing part guide hole 335 is connected to the second sheath guide hole 337 in which a first portion 344a of the second sheath 343 to be described below is disposed. In order to arrange the second sheath 343, one end portion, for example, a lower end portion based on FIG. 23 of the second sheath guide hole 337 is positioned adjacent to the sheath fixing part 334.

In this case, the second sheath guide hole 337 may be formed along a path curved at a predetermined curvature. In the present embodiment, a portion in which the second sheath guide hole 337 and the tendon fixing part guide hole 335 are connected may be formed in the circumferential direction of the body part 332, the lower end portion of the second sheath guide hole 337 may be formed in the longitudinal direction of the body part 332, and a portion positioned therebetween may be formed to be curved at a predetermined curvature.

The one sides 341 and 344 of the first and second sheaths 340 and 343 are fixedly coupled to the sheath fixing part 334. In the present embodiment, the one side 341 of the first sheath 340 may include the first portion 341a disposed inside the first sheath guide hole 336, and curved at the predetermined curvature and a second portion 341b formed to extend from one end portion of the first portion 341a in a longitudinal direction of the first movable member 330. The one side 344 of the second sheath 343 may include the first portion 344a disposed inside the second sheath guide hole 337, and curved at the predetermined curvature and a second portion 344b formed to extend from one end portion of the first portion 344a in the longitudinal direction of the first movable member 330.

Therefore, the one sides 341 and 344 of the first and second sheaths 340 and 343 can minimize a friction force and resistance which may be generated as the first and second tendons 320 and 323 passing through the inside of the moving device are curved, and guide the first and second tendons 320 and 323 in a circumferential direction of the first movable member 330.

Meanwhile, the other sides 342 and 345 of the first and second sheaths 340 and 343 are fixedly coupled to the first and second holders 370 and 376, respectively. **In** this case, the first and second holders 370 and 376 may be formed integrally.

The first tendon 320 passes through the first sheath 340 so that at least a portion thereof is disposed inside the first sheath 340. The one side 321 of the first tendon 320 is coupled to the tendon fixing part 314 of the stationary member 310.

**In** the present embodiment, the one side 321 of the first tendon 320 may include a first portion 321a of which one end is fixedly coupled to the tendon fixing part 314 and which extends in the circumferential direction of the first movable member 330 and a second portion extending from the first portion 321a, disposed inside the first portion 341a of the first sheath 340, and curved at a predetermined curvature. The other side 322 of the first tendon 320 is coupled to the first driving unit 380 to be pulled or released by the first driving unit 380.

Meanwhile, the one side 324 of the second tendon 323 may include a first portion 324a of which one end is fixedly coupled to the tendon fixing part 314 and which extends in the circumferential direction of the first movable member 330 and a second portion extending from the first portion 324a, disposed inside the first portion 344a of the second sheath 343, and curved at a predetermined curvature. The other side 325 of the second tendon 323 is coupled to the second driving unit 386 to be pulled or released by the second driving unit 386.

Hereinafter, a process in which the first movable member and the stationary member of the moving device according to the fourth embodiment not part of the present invention is rotated relative to each other by the first and second driving units will be described.

Referring to FIGS. 23 to 26, when the first driving unit 380 pulls the other side 322 of the first tendon 320, tension is applied to the first tendon 320, and the relative movement in which the other side 322 of the first tendon 320 is drawn out from the other side 342 of the first sheath 340 and the one side 321 of the first tendon 320 is inserted into the one side 341 of the first sheath 340 occurs. As the other side 322 of the first tendon 320 is pulled, the one side 321 of the first tendon 320 pulls the tendon fixing part 314 counterclockwise.

At the same time, an interaction force is generated between the first sheath 340 and the first tendon 320 by the relative movement between the first sheath 340 and the first tendon 320. In addition, since the other side 342 of the first sheath 340 with the predetermined compressive strength is supported by the first holder 370, a repulsive force is generated.

The generated repulsive force applies a force to the first movable member 330 through the sheath fixing part 334 fixed to the one side 341 of the first sheath 340. In this case, since the one side 321 of the first tendon 320 is disposed to be curved in the circumferential direction thereof and the one side 341 of the first sheath 340 is also disposed to be curved to allow an end portion to face a direction parallel to the circumferential direction, the generated repulsive force relatively rotates the first movable member 330 clockwise with respect to the stationary member 310.

Therefore, in the moving device 301 according to the present embodiment, the first driving unit 380 may relatively rotate the first movable member 330 clockwise with respect to the stationary member 310 by pulling the other side 322 of the first tendon 320.

At the same time, by smoothly causing the relative movement between the second sheath 343 and the second tendon 323 by an external force so that the other side 325 of the second tendon 323 may be slightly inserted into the other side 345 of the second sheath 343, the second driving unit 386 releases the other side 325 of the second tendon 323.

Therefore, since the resistance generated by the second tendon 323 and the second sheath 343 can be minimized, the first movable member 330 may be easily rotated with respect to the stationary member 310 by the relative movement between the first tendon 320 and the first sheath 340.

Meanwhile, when the second driving unit 386 pulls the other side 325 of the second tendon 323 and the first driving unit 380 releases the other side 322 of the first tendon 320, the first movable member 330 may be relatively rotated counterclockwise with respect to the stationary member 310 by an operating process similar to the above-described operating process.

As described above, in the moving device 301 according to the present embodiment, any one of the first and second driving units 380 and 386 may pull any one of the first and second tendons 320 and 323, and the other one of the first and second driving units 380 and 386 may release the other one of the first and second tendons 320 and 323 to relatively rotate the first movable member 330 clockwise or counterclockwise with respect to the stationary member 310.

Hereinafter, a moving device according to a fifth embodiment not part of of the present invention will be described.

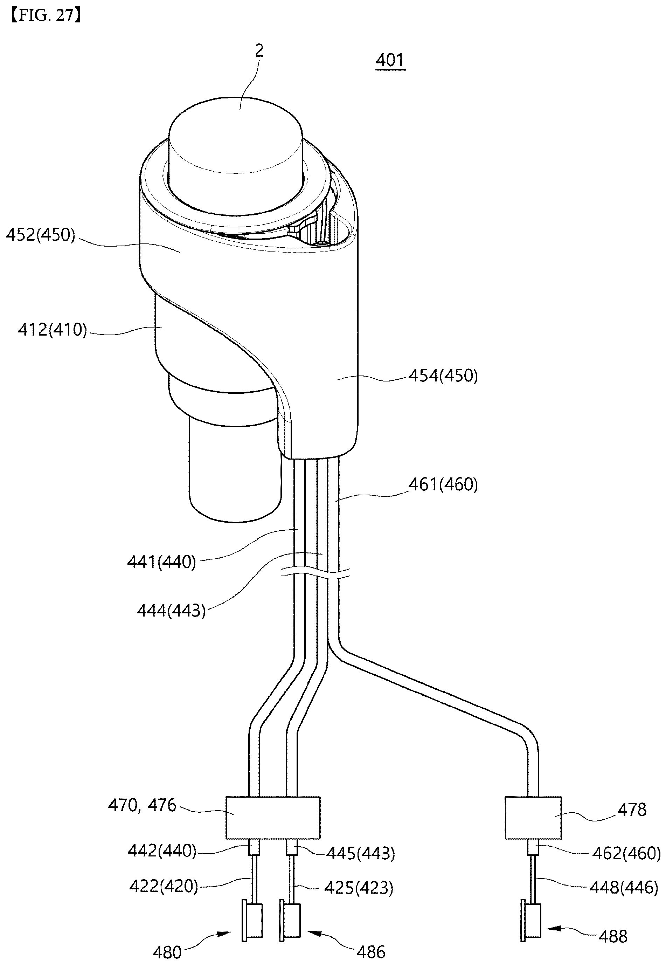

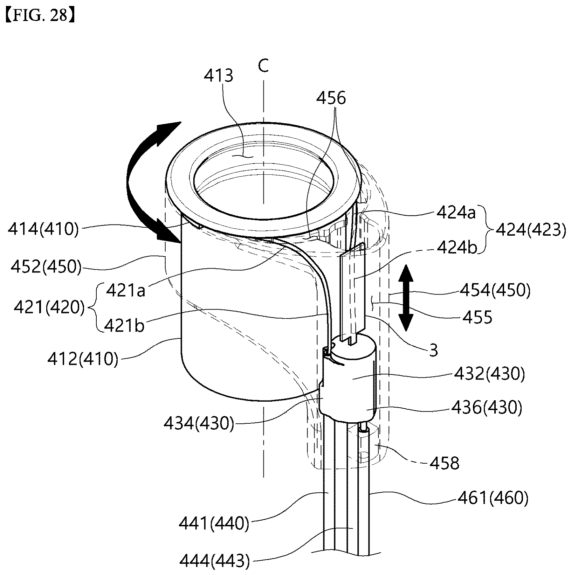

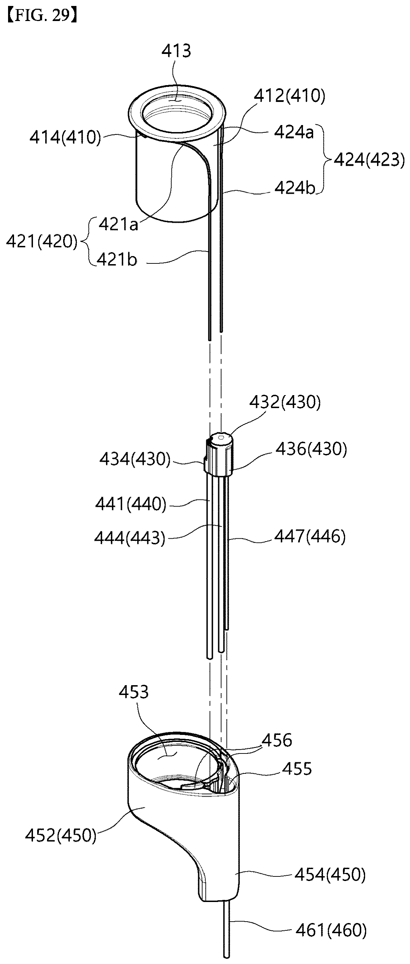

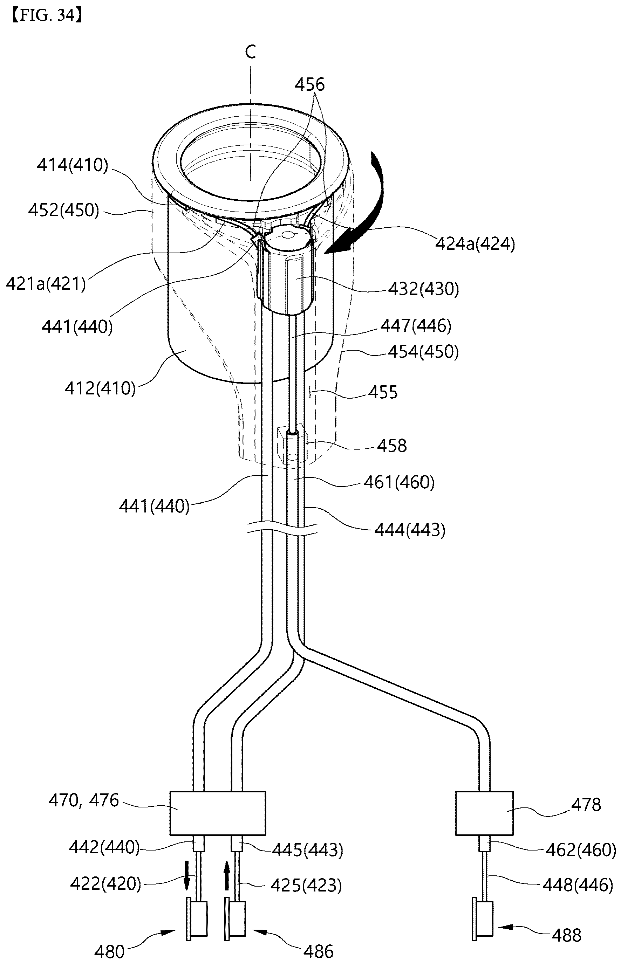

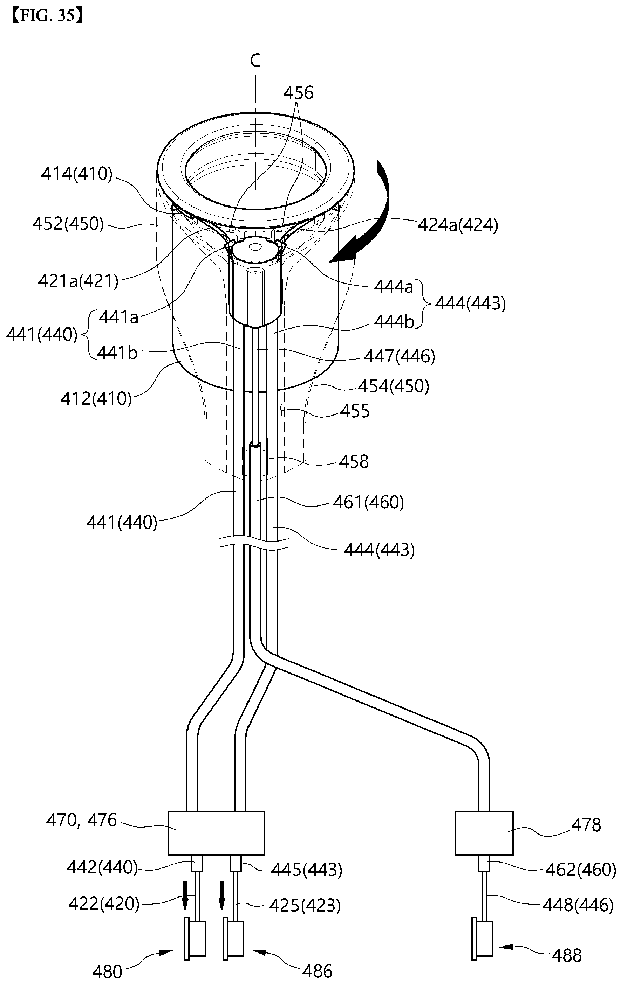

FIGS. 27 and 28 are schematic views illustrating a moving device according to a fifth embodiment not part of the present invention. FIG. 29 is an exploded perspective view of the moving device illustrated in FIG. 27. Hereinafter, in describing FIGS. 27 to 37, the description will be made by defining an upward direction of FIG. 27 as a forward direction, defining a downward direction as a rearward direction, and defining clockwise and counterclockwise based on when viewed from top. Meanwhile, in FIGS. 28 and 30 to 36, a second movable member is marked by dotted lines, and components projected inside the second movable member are marked by solid lines.

Referring to FIGS. 27 to 29, a moving device 401 according to a fifth embodiment not part of the present invention may include a stationary member 410, first and second tendons 420 and 423, a first movable member 430, first and second sheaths 440 and 443, a third tendon 446, a second movable member 450, and a third sheath 460.

The stationary member 410 may be configured as a body part 412, which is a cylindrical member having a coupling hole 413 formed at the center thereof. The tubular member, for example, the front end portion 2 of the endoscope device may be fixedly coupled to the coupling hole 413 of the body part 412. First and second tendon fixing parts 414 which are formed to protrude outward and to which one sides 421 and 424 of the first and second tendons 420 and 423 are fixedly coupled are formed on an outer circumferential surface of the stationary member 410.

More specifically, the one side 421 of the first tendon 420 may include a first portion 421a of which one end is coupled to one sides of the first and second tendon fixing parts 414 and which is formed to extend in a circumferential direction of the stationary member 410 and a second portion 421b formed to extend in a longitudinal direction of the body part 412 of the stationary member 410 from the first portion 421a.

In this case, a portion in which the first portion 421a and the second portion 421b of the first tendon 420 are connected is disposed to be curved at a predetermined curvature. Therefore, tension applied as the first tendon 420 is pulled by the first driving unit 280 to be described below may be smoothly converted into a force applied in the circumferential direction of the stationary member 410.

Similarly, the one side 424 of the first tendon 423 may include a first portion 424a of which one end is coupled to one sides of the first and second tendon fixing parts 414 and which is formed to extend in the circumferential direction of the stationary member 410 and a second portion 424b formed to extend in the longitudinal direction of the body part 412 of the stationary member 410 from the first portion 424a.

In this case, a portion in which the first portion 424a and the second portion 424b of the first tendon 423 are connected is disposed to be curved at a predetermined curvature. Therefore, tension applied as the second tendon 423 is pulled by the second driving unit 286 to be described below may be smoothly converted into a force applied in the circumferential direction of the stationary member 410.

The second movable member 450 may be formed in a cylindrical shape and may include a body part 452 having a coupling hole 453 formed in a central portion thereof and an accommodating part 454 formed on an outer side portion of the body part 452. The accommodating part 454 may be formed to extend in a longitudinal direction (or in a front-rear direction) of the body part 452. A first guide hole 455 to which the first movable member 430 to be described below is coupled to move relatively is formed in the accommodating part 454.

The body part 412 of the stationary member 410 and the body part 452 of the second movable member 450 may be coaxially arranged about the central axis C. In this case, the stationary member 410 may be coupled to the coupling hole 453 of the body part 452 of the second movable member 450 to rotate relatively about the central axis C and not to move relatively in a direction of the central axis C.

Meanwhile, the first guide hole 455 of the accommodating part 454 is formed to extend in a longitudinal direction of the body part 452. In this case, a third sheath fixing part 458 to which one side 461 of the third sheath 460 to be described below is fixedly coupled is formed on one end portion, for example, a rear end portion based on FIG. 27 of the first guide hole 455. The third sheath fixing part 458 may be configured as a fixing hole to which the third sheath 460 is insertion-coupled, but is not limited thereto, and may be configured in various configurations which may be fixed to restrict the relative movement of the third sheath 460.

A second guide hole 456, which has an area of a cross section perpendicular to the longitudinal direction, which increases forward and surrounds an outer circumferential portion of the stationary member 410 in a circumferential direction, may be formed on the other end portion, for example, a front end portion based on FIG. 27 of the first guide hole 455.

In this case, a portion in which the first guide hole 455 and the second guide hole 456 are connected may be formed to be inclined in directions in which one side surface and the other side surface in the circumferential direction are away from each other and formed to be slightly rounded.

The first portion 421a of the first tendon 420, the first portion 424a of the second tendon 423, and the first and second tendon fixing parts 414 are disposed inside the second guide hole 456 surrounding the outer circumferential portion of the stationary member 410. The second portion 421b of the first tendon 420 and the second portion 424b of the second tendon 423 are disposed on the portion in which the first guide hole 455 and the second guide hole 456 are connected.

As described above, the one sides 421 and 424 of the first and second tendons 420 and 423 may be guided by the first and second guide holes 455 and 456 and disposed to maintain a smoothly curved shape.

The first movable member 430 is coupled to the first and second guide holes 455 and 456 to relatively move in an extending direction of the accommodating part 454. However, the first movable member 430 is coupled to the second movable member 450 to restrict the rotation relative to the second movable member 450 about the central axis C.

The first movable member 430 may be configured as a body part 432 with a predetermined length. A first sheath fixing part 434 to which one side 441 of the first sheath 440 is coupled and a second sheath fixing part 435 to which one side 444 of the second sheath 443 is coupled are formed on one side portion of the first movable member. The surgical member 3 for an endoscope device may be provided on the one side portion of the first movable member 430.

More specifically, a first portion 441a of the first sheath 440, which may have a predetermined degree of freedom and may be curved, is positioned at one side, for example, an upper side based on FIG. 27 of the first sheath fixing part 434. A second portion 441b extending from the first portion 441a is coupled to the first sheath fixing part 434. Since the first portion 441a of the first sheath 440 is not restricted by the first sheath fixing part 434 and has predetermined variability, the first portion 441a may be curved outward from the first movable member 430.

Similarly, a first portion 444a of the second sheath 443, which may have a predetermined degree of freedom and may be curved, is positioned at one side, for example, an upper side based on FIG. 27 of the second sheath fixing part 435. A second portion 444b extending from the first portion 444a is coupled to the second sheath fixing part 435. Since the first portion 444a of the second sheath 443 is not restricted by the second sheath fixing part 435 and has predetermined variability, the first portion 444a may be curved outward from the first movable member 430.

Functions performed by the first portions 441a and 444a of the first and second sheaths 440 and 443 will be described below in relation to the relative movement between the first movable member 430 and the second movable member 450.

One side 447 of the third tendon 446 is fixedly coupled to the other side portion, for example, a rear side portion based on FIG. 27 of the first movable member 430. At least a portion of the third tendon 446 may pass through the third sheath 460, and the one side 447 of the third tendon 446 may be drawn out from the one side 461 of the third sheath 460.

At least a portion of the first tendon 420 may pass through the first sheath 440, and the one side 421 of the first tendon 420 may be drawn out from the one side 441 of the first sheath 440. At least a portion of the second tendon 423 may pass through the second sheath 443, and the one side 424 of the second tendon 423 may be drawn out from the one side 444 of the second sheath 443.

In other words, the first and second tendon fixing parts 414 to which the one sides 421 and 424 of the first and second tendons 420 and 423 are fixedly coupled are positioned at the front of the first movable member 430, and the third sheath fixing part 458 to which the one side 461 of the third sheath 460 is fixedly coupled is positioned at the rear of the first movable member 430.

Meanwhile, the other sides 442, 445, and 448 of the first, second, and third sheaths 440, 443, and 460 are fixedly coupled to the first, second, and third holders 470, 476, and 478, respectively, and the other sides 422, 425, and 448 of the first, second, and third tendons 420, 423, and 446 are coupled to the first, second, and third driving units 480, 486, and 488, respectively.

When the first, second, and third driving units 480, 486, and 488 pull the other sides 422, 425, and 448 of the first, second, and third tendons 420, 423, and 446, the other sides 442, 445, and 448 of the first, second, and third sheaths 440, 443, and 460 are fixed by the first, second, and third holders 470, 476, and 478, respectively, and thus the other sides 422, 425, and 448 of the first, second, and third tendons 420, 423, and 446 may be drawn out from the other sides 442, 445, and 448 of the first, second, and third sheaths 440, 443, and 460, thereby causing the relative movement between the first, second, and third tendons 420, 423, and 446 and the first, second, and third sheaths 440, 443, and 460.

Hereinafter, a process in which the stationary member, the first movable member, and the second movable member of the moving device according to the fifth embodiment are relatively moved by the first to third holders and the first to third driving units will be described.

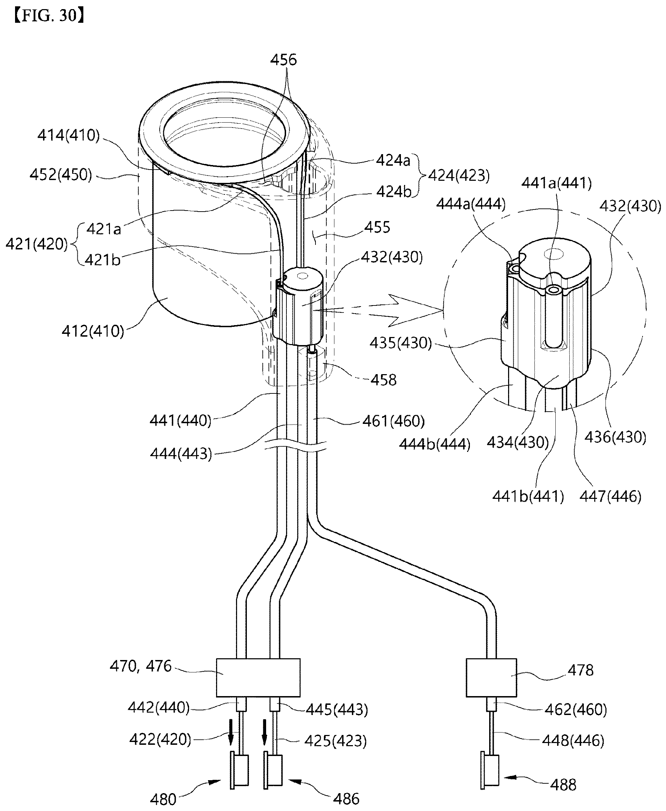

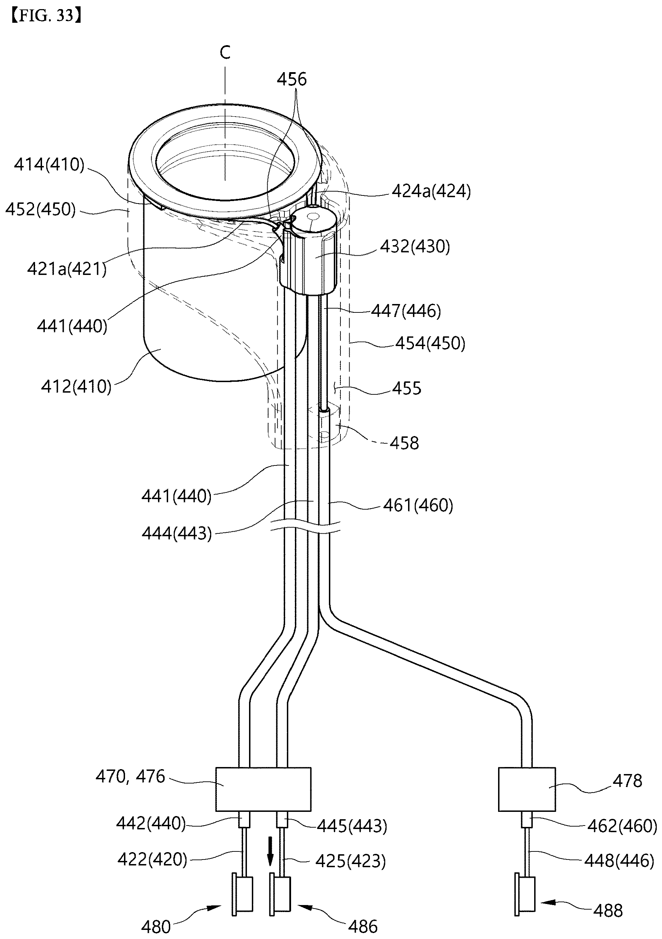

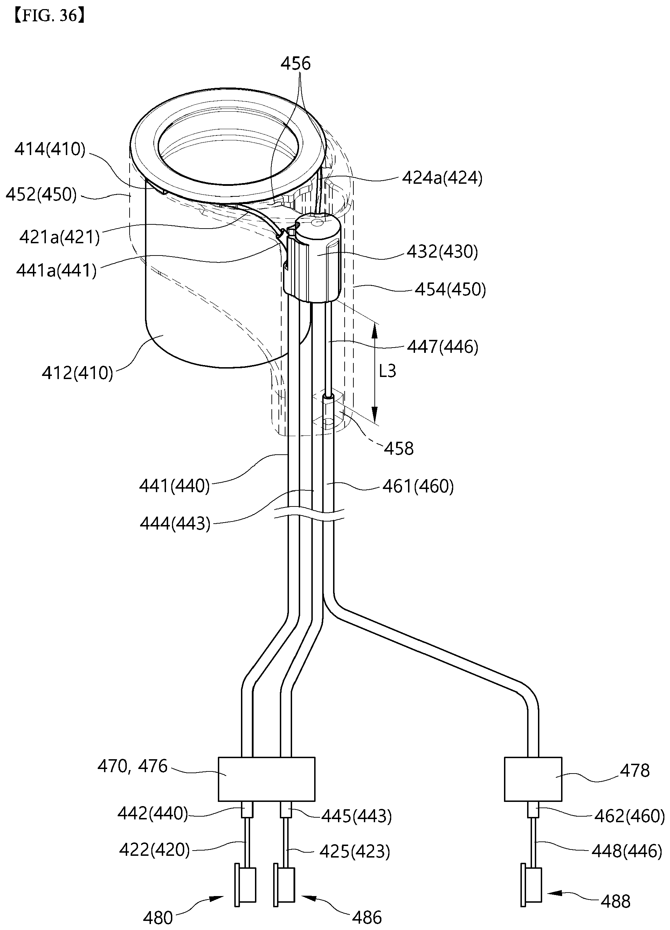

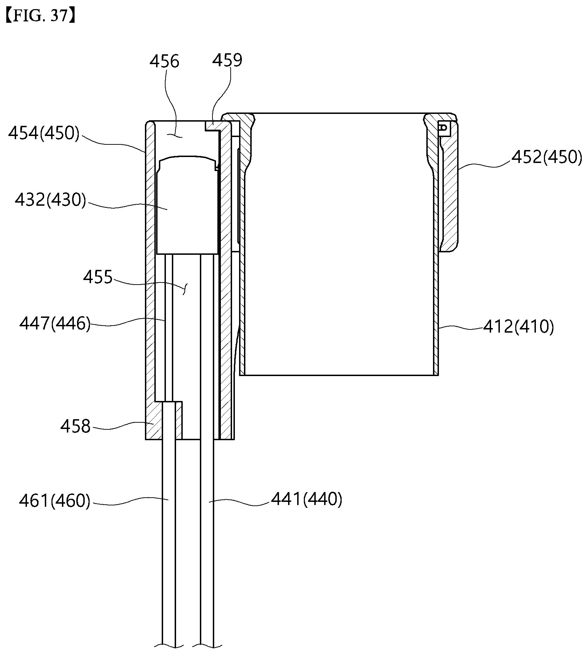

FIGS. 30 to 32 are views for describing a process in which a first movable member of the moving device illustrated in FIG. 27 relatively moves with respect to a stationary member and a second movable member. FIGS. 33 to 35 are views for describing a process in which the first movable member and the second movable member of the moving device illustrated in FIG. 27 relatively moves with respect to the stationary member. FIG. 36 is a view for describing one example of a method of operating the first movable member illustrated in FIG. 27. FIG. 37 is a cross-sectional view of the stationary member, the first movable member, and the second movable member illustrated in FIG. 27.

Referring to FIGS. 30 to 32, in the present embodiment, the first movable member 430 may relatively move in a front-rear direction with respect to the stationary member 410 and the second movable member 450. In this case, the relative movement of the first movable member 430 is caused by the relative movement between the first and second tendons 420 and 423 and the first and second sheaths 440 and 443 and the relative movement between the third tendon 446 and the third sheath 460.

The linear relative movement of the first movable member 430 according to one embodiment may be performed in the same process as that of any one of the moving devices 1, 101, and 201 (see FIGS. 1, 10, and 16) according to the first to third embodiments.

More specifically, in the present embodiment, the linear relative movement of the first movable member 430 may be performed in the same process as that of the moving device 101 (see FIG. 10) according to the second embodiment. In other words, the stationary member 410 and the second movable member 450 may be configured to perform the same function as that of the stationary member 110 (see FIG. 10) of the second embodiment, the first movable member 430 may be configured to perform the same function as that of the first movable member 130 (see FIG. 10) of the second embodiment, the first and second tendons 420 and 430 may be configured to perform the same function as that of the first tendon 120 (see FIG. 10) of the second embodiment, the first and second sheaths 440 and 443 may be configured to perform the same function as that of the first sheath 140 (see FIG. 10) of the second embodiment, the third tendon 446 may be configured to perform the same function as that of the second tendon 143 (see FIG. 10) of the second embodiment, the third sheath 460 may be configured to perform the same function as that of the second sheath 123 (see FIG. 10) of the second embodiment, the first and second holders 470 and 476 may be configured to perform the same functions as that of the first holder 170 (see FIG. 10) of the second embodiment, the third holder 478 may be configured to perform the same function as that of the second holder 176 (see FIG. 10) of the second embodiment, the first and second driving units 480 and 486 may be configured to perform the same functions as that of the first driving unit 180 (see FIG. 10) of the second embodiment, and the third driving unit 488 may be configured to perform the same function as that of the second driving unit 186 (see FIG. 10) of the second embodiment.

Referring to FIG. 30, when the first and second driving units 480 and 486 pull the other sides 422 and 425 of the first and second tendons 420 and 423, respectively, the other sides 422 and 425 of the first and second tendons 420 and 423 may be drawn out from the other sides 442 and 445 of the first and second sheaths 440 and 443 fixedly coupled to the first and second holders 470 and 476, thereby causing the relative movement between the first and second tendons 420 and 423 and the first and second sheaths 440 and 443.

Meanwhile, the third driving unit 488 releases the other side 448 of the third tendon 446 and provides a predetermined degree of freedom to cause relative movement between the third tendon 446 and the third sheath 460.

In this case, when the first movable member 430 is positioned on a rear portion of the first guide hole 455, the first portions 441a and 444a of the first and second sheaths 440 and 443 are disposed parallel to the extending direction of the first guide hole 455 or the longitudinal direction of the stationary member 410. Hereinafter, the position of the first movable member 430 is referred to as a second position.

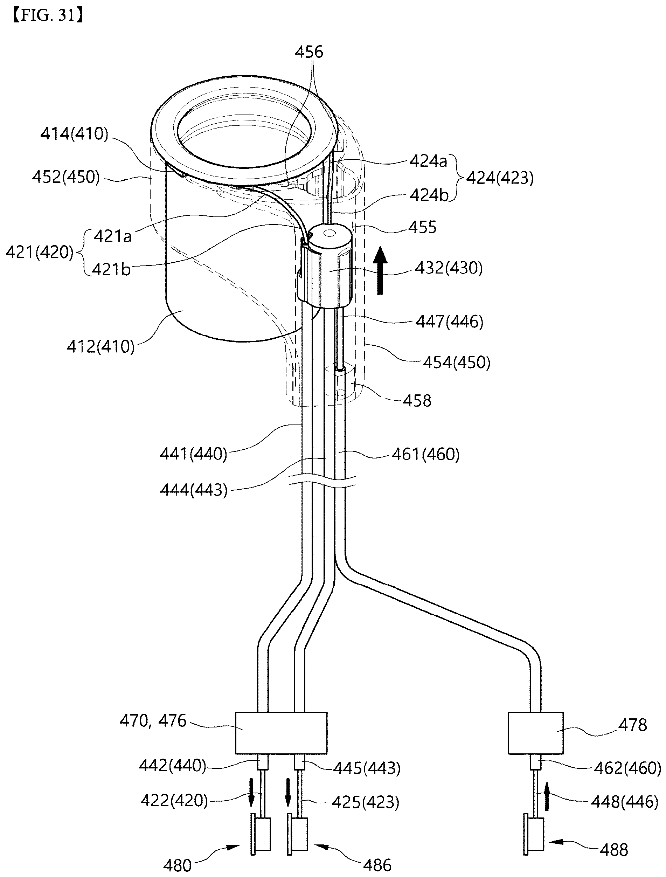

Referring to FIG. 31, when the other sides 421 and 424 of the first and second tendons 420 and 423 are pulled rearward by the first and second driving units 480 and 486, as described above in the second embodiment, the first and second sheaths 440 and 443 have predetermined compressive strengths and are supported by the first and second holders 470 and 476, thereby generating a repulsive force pushing the first movable member 430 forward.

As described above, according to the present embodiment, the first movable member 430 may perform the linear relative movement forward with respect to the stationary member 410 and the second movable member 450 by the relative movement between the first and second tendons 420 and 423 and the first and second sheaths 440 and 443.

In this case, since the third driving unit 488 releases the other side 448 of the third tendon 446, the other side 448 of the third tendon 446 may be inserted into the third sheath 460. Therefore, the first movable member 430 may move forward with less resistance generated by the third tendon 446.

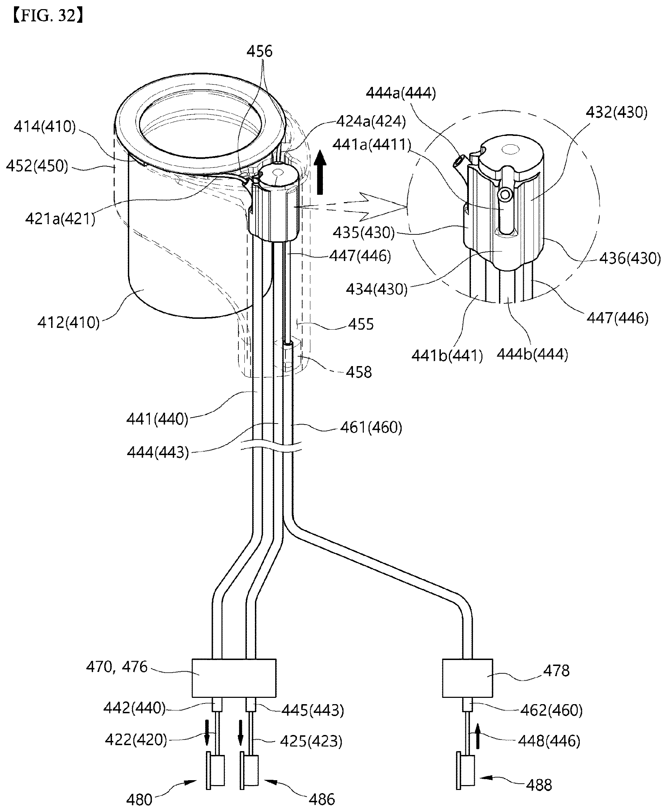

Referring to FIG. 32, when the first movable member 430 reaches a front side of the first guide hole 455, the relative movement between the first movable member 430 and the second movable member 450 may be restricted to prevent the first movable member 430 from exiting to the outside of the accommodating part 454 of the second movable member 450. The above action may be implemented by a stopper provided on the second movable member 450. The stopper will be described below with reference to FIG. 37.

Meanwhile, as the first movable member 430 moves forward from the first guide hole 455, when the first portions 441a and 444a of the first and second sheaths 440 and 443 enter the second guide hole 456, the first portions 441a and 444a of the first and second sheaths 440 and 443 are curved to the outside of the first movable member 430.

In this case, each of the first portions 441a and 444a may be disposed to be curved at a predetermined curvature along an inner surface positioned on one of both side portions of the second guide hole 456 in the circumferential direction. Therefore, the first portions 441a and 444a of the first and second sheaths 440 and 443 can minimize a friction force and resistance generated between the first and second tendons 420 and 423 and guide the first and second tendons 420 and 423 extending in the circumferential direction of the stationary member 410.

Referring to FIGS. 33 to 35, in the present embodiment, the first movable member 430 and the second movable member 450 may relatively rotate clockwise or counterclockwise with respect to the stationary member 410 about the central axis C. In this case, the relative rotation between the first movable member 430 and the second movable member 450 may be caused by the relative movement between the first tendon 420 and the first sheath 440 and the relative movement between the second tendon 423 and the second sheath 443.

In the present embodiment, the relative rotation between the first movable member 430 and the second movable member 450 may be performed in the same process as that of the moving device 301 (see FIG. 23) according to the fourth embodiment.

More specifically, the stationary member 410 may be configured to perform the same function as that of the stationary member 310 (see FIG. 23) of the fourth embodiment, the first movable member 430 and the second movable member 450 may be configured to perform the same functions as that of the first movable member 430 (see FIG. 23) of the fourth embodiment, the second guide hole 456 may be configured to perform the same function as that of the tendon fixing part guide hole 335 (see FIG. 23) of the fourth embodiment, the first and second tendon fixing parts 414 may be configured to perform the same function as that of the tendon fixing part 314 (see FIG. 23) of the fourth embodiment, the first and second tendons 420 and 423 may be configured to perform the same functions as those of the first and second tendons 320 and 323 (see FIG. 23) of the fourth embodiment, the first and second sheaths 440 and 443 may be configured to perform the same functions as those of the first and second sheaths 340 and 343 (see FIG. 23) of the fourth embodiment, the first and second holders 470 and 476 may be configured to perform the same functions as those of the first and second holders 370 and 376 of the fourth embodiment, and the first and second driving units 480 and 486 may be configured to perform the same functions as those of the first and second driving units 380 and 386 of the fourth embodiment.

Referring to FIGS. 33 to 35, when the first driving unit 480 pulls the other side 422 of the first tendon 420, tension is applied to the first tendon 420, and the relative movement in which the other side 422 of the first tendon 420 is drawn out from the other side 442 of the first sheath 440 occurs. As the other side 422 of the first tendon 420 is pulled, the one side 421 of the first tendon 420 pulls the tendon fixing part 414 counterclockwise.

At the same time, an interaction force is generated between the first sheath 440 and the first tendon 420 by the relative movement between the first sheath 440 and the first tendon 420. In addition, since the first sheath 440 with the predetermined compressive strength is supported by the first holder 470, a repulsive force is generated forward.

The generated repulsive force applies a force to the first movable member 430 through the first and second sheath fixing parts 434 and 435 fixed to the one side 441 of the first sheath 440. In this case, since the first portion 421a of the first tendon 420 is disposed to be curved in the circumferential direction thereof and the first portion 441a of the first sheath 440 is also disposed to be curved to allow an end portion to face a direction parallel to the circumferential direction, the generated repulsive force relatively rotates the first movable member 430 and the second movable member 450 clockwise with respect to the stationary member 410.

Therefore, in the moving device 401 according to the present embodiment, the first driving unit 480 may relatively rotate the first movable member 430 and the second movable member 450 clockwise with respect to the stationary member 410 by pulling the other side 422 of the first tendon 420.

At the same time, in order to smoothly cause the relative movement between the second sheath 443 and the second tendon 423 by an external force, the second driving unit 486 releases the other side 425 of the second tendon 423.

Therefore, since the resistance generated by the second tendon 423 and the second sheath 443 can be minimized, the first movable member 330 and the second movable member 450 may be easily rotated with respect to the stationary member 410 by the relative movement between the first tendon 420 and the first sheath 440.

Meanwhile, when the second driving unit 486 pulls the other side 425 of the second tendon 423 and the first driving unit 480 releases the other side 422 of the first tendon 420, the first movable member 430 and the second movable member 450 may be relatively rotated counterclockwise with respect to the stationary member 410 by an operating process similar to the above-described operating process.

As described above, in the moving device 401 according to the present embodiment, since any one of the first and second driving units 480 and 486 pulls any one of the first and second tendons 420 and 423, and the other one of the first and second driving units 480 and 486 releases the other one of the first and second tendons 420 and 423, the first movable member 430 and the second movable member 450 are relatively rotated clockwise or counterclockwise with respect to the stationary member 410.

As described above, according to the present embodiment, it is possible to linearly relatively move or rotate the first movable member 430 with respect to the stationary member 410 using the relative movement between the first to third tendons 420, 423, and 446 and the first to third sheaths 440, 443, and 460.

Meanwhile, the user who uses the moving device according to one embodiment may fix the relative positions between the first movable member and the second movable member in the process in which the first movable member and the second movable member relatively rotate with respect to the stationary member.

Referring to FIG. 36, in the present embodiment, the above-described operation may be performed by controlling the third driving unit 488. More specifically, in order to constantly maintain a distance L3 between a rear end portion of the first movable member 430 and a rear end portion of the first guide hole 455, the user may fix the relative positions between the first movable member 430 and the second movable member 450 by pulling rearward, fixing a position of, or releasing the other side 448 of the third tendon 446 by the third driving unit 488.

Referring to FIG. 37, a stopper 459 protruding inward may be formed on front end portions of the first and second guide holes 445 and 446. The first movable member 430 moving forward in the first and second guide holes 445 and 446 is caught by the stopper 459 to be restricted forward movement thereof.

As described above, since the stopper 459 prevents the first movable member 430 from exiting to the outside of the accommodating part 454 of the second movable member 450 by restricting the forward movement of the first movable member 430, the first movable member 430 may be accommodated inside the accommodating part 454 and protected from the outside.

In addition, the stopper 459 may serve to restrict a distance of the first movable member 430 linearly relatively moving forward with respect to the second movable member 450. In other words, the linear movement distance of the first movable member 430 is restricted to a section between the third sheath fixing part 458 and the stopper 459 formed in the accommodating part 454.

In the present embodiment, although the stopper 459 is positioned at the front end portions of the first and second guide holes 455 and 456, the stopper 459 is positioned on central portions of the first and second guide holes 455 and 456 to restrict the linear movement distance of the first movable member 430 to be shorter slightly.

The relative functions or relative movements of the stationary member and the first movable member or the second movable member described throughout the specification may be variously modified or changed without departing from the present invention.

For example, in relation to the second embodiment of the present invention, although it has been described in FIG. 10 that reference numeral 110 indicates the stationary member to which the first tendon 120 and the second sheath 123 is coupled, reference numeral 130 indicates the first movable member, and when the second tendon 143 and the first sheath 140 on which the curved portion 140a is formed are coupled to the first movable member and the first tendon 120 is pulled rearward by the first driving unit 180, the first movable member 130 is relatively moved forward with respect to the stationary member 110 by the compressive force and repulsive force of the first sheath 140, and in another embodiment, in FIG. 10, reference numeral 110 indicates the first movable member, reference numeral 130 indicates the stationary member, and when the second driving unit 186 pulls the second tendon 143 rearward, a part indicated by the reference numeral 110 may be relatively moved forward with respect to a part indicated by the reference numeral 130 by the compressive force and repulsive force of the second sheath 123. In this case, it will be preferable that a predetermined curved portion be formed on the second sheath 123 to generate a repulsive force and a compressive force.

## Claims

1. A moving device comprising:
a stationary member (10);
a first tendon (20) of which one side is coupled to the stationary member;
a first movable member (30) that is movable with respect to the stationary member;
a first sheath (40) of which one side is coupled to the first movable member and which is formed to allow the first tendon to pass therethrough;
a first holder (70) configured to fix any one of the other side of the first tendon and the other side of the first sheath; and
a first driving unit (80) configured to move the other one of the other side of the first tendon and the other side of the first sheath,
wherein the first movable member is formed to relatively move with respect to the stationary member according to relative movement of the first tendon and the first sheath,
**characterized in that** the moving device further comprises:
a second tendon (43) of which one side is coupled to the first movable member;
a second sheath (23) of which one side is coupled to the stationary member;
a second holder (76) configured to fix any one of the other side of the second tendon and the other side of the second sheath; and
a second driving unit (86) configured to move the other one of the other side of the second tendon and the other side of the second sheath,
wherein the first movable member is formed to relatively move with respect to the stationary member according to relative movement of the second tendon and the second sheath.

2. The moving device of claim 1, wherein the first holder is formed to fix the other side of the first sheath, and
the first driving unit is formed to move the other side of the first tendon.

3. The moving device of claim 2, wherein, when the first driving unit pulls the other side of the first tendon, relative movement between the stationary member and the first movable member is caused as the one side of the first tendon is inserted into the one side of the first sheath, and the other side of the first tendon is drawn out from the other side of the first sheath.

4. The moving device of claim 1, wherein the first holder is formed to fix the other side of the first tendon, and
the first driving unit is formed to move the other side of the first sheath.

5. The moving device of claim 1, wherein the first driving unit includes an actuator or an operating member manipulated manually.

6. The moving device of claim 1, wherein the first driving unit includes a guide member configured to guide the other one of the other side of the first tendon and the other side of the first sheath.

7. The moving device of claim 1, wherein the second sheath is formed to allow the second tendon to pass therethrough, and wherein the first movable member is formed to relatively move with respect to the stationary member between the one side of the first tendon coupled to the stationary member and the one side of the second sheath coupled to the stationary member.

8. The moving device of claim 1, wherein the first tendon and the second tendon are disposed parallel to each other in a direction of relative movement between the first movable member and the stationary member.

9. The moving device of claim 1, wherein the one side of the first tendon coupled to the stationary member is positioned in front of the first movable member when viewed in a moving direction of the first movable member, and
the one side of the second sheath coupled to the stationary member is positioned behind the first movable member when viewed in the moving direction of the first movable member.

10. The moving device of claim 1, wherein the stationary member is fixedly positioned on a front end portion of an endoscope device.

11. The moving device of claim 1, wherein a surgical member for an endoscope device is coupled to the first movable member.

## Patentansprüche

1. Bewegungsvorrichtung, umfassend:
ein feststehendes Element (10);
eine erste Sehne (20), deren eine Seite mit dem feststehenden Element gekoppelt ist;
ein erstes bewegliches Element (30), das in Bezug auf das feststehende Element beweglich ist;
eine erste Hülle (40), deren eine Seite mit dem ersten beweglichen Element gekoppelt ist und die so ausgebildet ist, dass die erste Sehne durch sie hindurchtreten kann;
einen ersten Halter (70), der dazu ausgebildet ist, eine von der anderen Seite der ersten Sehne und der anderen Seite der ersten Hülle zu fixieren; und
eine erste Antriebseinheit (80), die dazu ausgebildet ist, die jeweils andere von der anderen Seite der ersten Sehne und der anderen Seite der ersten Hülle zu bewegen,
wobei das erste bewegliche Element dazu ausgebildet ist, sich entsprechend einer Relativbewegung der ersten Sehne und der ersten Hülle relativ in Bezug auf das feststehende Element zu bewegen,
**dadurch gekennzeichnet, dass** die Bewegungsvorrichtung ferner Folgendes umfasst:
eine zweite Sehne (43), deren eine Seite mit dem ersten beweglichen Element gekoppelt ist;
eine zweite Hülle (23), deren eine Seite mit dem feststehenden Element gekoppelt ist;
einen zweiten Halter (76), der dazu ausgebildet ist, eine von der anderen Seite der zweiten Sehne und der anderen Seite der zweiten Hülle zu fixieren; und
eine zweite Antriebseinheit (86), die dazu ausgebildet ist, die jeweils andere von der anderen Seite der zweiten Sehne und der anderen Seite der zweiten Hülle zu bewegen,
wobei das erste bewegliche Element dazu ausgebildet ist, sich entsprechend einer Relativbewegung der zweiten Sehne und der zweiten Hülle relativ in Bezug auf das feststehende Element zu bewegen.

2. Bewegungsvorrichtung nach Anspruch 1, wobei der erste Halter dazu ausgebildet ist, die andere Seite der ersten Hülle zu fixieren, und
die erste Antriebseinheit dazu ausgebildet ist, die andere Seite der ersten Sehne zu bewegen.

3. Bewegungsvorrichtung nach Anspruch 2, wobei, wenn die erste Antriebseinheit an der anderen Seite der ersten Sehne zieht, eine Relativbewegung zwischen dem feststehenden Element und dem ersten beweglichen Element hervorgerufen wird, indem die eine Seite der ersten Sehne in die eine Seite der ersten Hülle eingeführt wird und die andere Seite der ersten Sehne aus der anderen Seite der ersten Hülle herausgezogen wird.

4. Bewegungsvorrichtung nach Anspruch 1, wobei der erste Halter dazu ausgebildet ist, die andere Seite der ersten Sehne zu fixieren, und
die erste Antriebseinheit dazu ausgebildet ist, die andere Seite der ersten Hülle zu bewegen.

5. Bewegungsvorrichtung nach Anspruch 1, wobei die erste Antriebseinheit einen Aktuator oder ein manuell betätigtes Bedienelement aufweist.

6. Bewegungsvorrichtung nach Anspruch 1, wobei die erste Antriebseinheit ein Führungselement aufweist, das dazu ausgebildet ist, die jeweils andere von der anderen Seite der ersten Sehne und der anderen Seite der ersten Hülle zu führen.

7. Bewegungsvorrichtung nach Anspruch 1, wobei die zweite Hülle so ausgebildet ist, dass die zweite Sehne durch sie hindurchtreten kann, und wobei das erste bewegliche Element dazu ausgebildet ist, sich zwischen der mit dem feststehenden Element gekoppelten einen Seite der ersten Sehne und der mit dem feststehenden Element gekoppelten einen Seite der zweiten Hülle relativ in Bezug auf das feststehende Element zu bewegen.

8. Bewegungsvorrichtung nach Anspruch 1, wobei die erste Sehne und die zweite Sehne in einer Richtung der Relativbewegung zwischen dem ersten beweglichen Element und dem feststehenden Element parallel zueinander angeordnet sind.

9. Bewegungsvorrichtung nach Anspruch 1, wobei die mit dem feststehenden Element gekoppelte eine Seite der ersten Sehne in einer Bewegungsrichtung des ersten beweglichen Elements gesehen vor dem ersten beweglichen Element positioniert ist, und
die mit dem feststehenden Element gekoppelte eine Seite der zweiten Hülle in der Bewegungsrichtung des ersten beweglichen Elements gesehen hinter dem ersten beweglichen Element positioniert ist.

10. Bewegungsvorrichtung nach Anspruch 1, wobei das feststehende Element ortsfest an einem vorderen Endabschnitt einer Endoskopvorrichtung positioniert ist.

11. Bewegungsvorrichtung nach Anspruch 1, wobei ein chirurgisches Element für eine Endoskopvorrichtung mit dem ersten beweglichen Element gekoppelt ist.

## Revendications

1. Dispositif de déplacement comprenant :
un élément fixe (10) ;
un premier tendon (20) dont un côté est couplé à l'élément fixe ;
un premier élément mobile (30) qui est mobile par rapport à l'élément fixe ;
une première gaine (40) dont un côté est couplé au premier élément mobile et qui est formée pour permettre au premier tendon de passer à travers celle-ci ;
un premier organe de maintien (70) configuré pour fixer l'un quelconque parmi l'autre côté du premier tendon et l'autre côté de la première gaine ; et
une première unité d'entraînement (80) configurée pour déplacer l'autre parmi l'autre côté du premier tendon et l'autre côté de la première gaine,
dans lequel le premier élément mobile est formé pour se déplacer relativement par rapport à l'élément fixe en fonction d'un mouvement relatif du premier tendon et de la première gaine,
**caractérisé en ce que** le dispositif de déplacement comprend en outre :
un deuxième tendon (43) dont un côté est couplé au premier élément mobile ;
une deuxième gaine (23) dont un côté est couplé à l'élément fixe ;
un deuxième organe de maintien (76) configuré pour fixer l'un quelconque parmi l'autre côté du deuxième tendon et l'autre côté de la deuxième gaine ; et
une deuxième unité d'entraînement (86) configurée pour déplacer l'autre parmi l'autre côté du deuxième tendon et l'autre côté de la deuxième gaine,
dans lequel le premier élément mobile est formé pour se déplacer relativement par rapport à l'élément fixe en fonction d'un mouvement relatif du deuxième tendon et de la deuxième gaine.

2. Dispositif de déplacement selon la revendication 1, dans lequel le premier organe de maintien est formé pour fixer l'autre côté de la première gaine, et
la première unité d'entraînement est formée pour déplacer l'autre côté du premier tendon.

3. Dispositif de déplacement selon la revendication 2, dans lequel, lorsque la première unité d'entraînement tire l'autre côté du premier tendon, un mouvement relatif entre l'élément fixe et le premier élément mobile est provoqué à mesure que ledit un côté du premier tendon est inséré dans ledit un côté de la première gaine et que l'autre côté du premier tendon est extrait de l'autre côté de la première gaine.

4. Dispositif de déplacement selon la revendication 1, dans lequel le premier organe de maintien est formé pour fixer l'autre côté du premier tendon, et
la première unité d'entraînement est formée pour déplacer l'autre côté de la première gaine.

5. Dispositif de déplacement selon la revendication 1, dans lequel la première unité d'entraînement comprend un actionneur ou un élément de commande actionné manuellement.

6. Dispositif de déplacement selon la revendication 1, dans lequel la première unité d'entraînement comprend un élément de guidage configuré pour guider l'autre parmi l'autre côté du premier tendon et l'autre côté de la première gaine.

7. Dispositif de déplacement selon la revendication 1, dans lequel la deuxième gaine est formée pour permettre au deuxième tendon de passer à travers celle-ci, et dans lequel le premier élément mobile est formé pour se déplacer relativement par rapport à l'élément fixe entre ledit un côté du premier tendon couplé à l'élément fixe et ledit un côté de la deuxième gaine couplé à l'élément fixe.

8. Dispositif de déplacement selon la revendication 1, dans lequel le premier tendon et le deuxième tendon sont disposés parallèlement l'un à l'autre dans une direction de mouvement relatif entre le premier élément mobile et l'élément fixe.

9. Dispositif de déplacement selon la revendication 1, dans lequel ledit un côté du premier tendon couplé à l'élément fixe est positionné devant le premier élément mobile, vu dans une direction de déplacement du premier élément mobile, et
ledit un côté de la deuxième gaine couplé à l'élément fixe est positionné derrière le premier élément mobile, vu dans la direction de déplacement du premier élément mobile.

10. Dispositif de déplacement selon la revendication 1, dans lequel l'élément fixe est positionné de manière fixe sur une partie d'extrémité avant d'un dispositif d'endoscope.

11. Dispositif de déplacement selon la revendication 1, dans lequel un élément chirurgical pour un dispositif d'endoscope est couplé au premier élément mobile.
